# EUROPEAN PATENT APPLICATION

(11) **EP 3 369 434 A1**
(43) Date of publication of application: **05.09.2018**
(21) Application number: 16859921.5
(22) Date of filing: 27.10.2016
(51) Int. Cl.: A61K 45/06, A61K 31/713, A61K 48/00, A61P 35/00, A61P 43/00, C12N 5/0775, C12N 5/09, C12N 15/00

(54) **INHIBITORS FOR METHYLATION-RELATED ENZYMES HAT1 AND KAT8**

(30) Priority: 30.10.2015 JP 2015215267
(71) Applicant: MIURA, Norimasa, Tottori 6830845 (JP)
(72) Inventor: MIURA, Norimasa, Tottori 6830845 (JP)
(74) Representative: Gulde & Partner
(86) International application number: PCT/JP2016/081939
(87) International publication number: WO 2017/073692

(57) **Abstract**

The purpose of the present invention is to obtain novel therapeutic drugs for malignant tumors, methods for producing a stem cell, or DNA damage-ameliorating agents, etc., wherein a HAT1 and KAT8 inhibitor is used; a kit containing a HAT1 inhibitor and a KAT8 inhibitor may be used; a composition comprising a HAT1 inhibitor, wherein the HAT1 inhibitor is used in combination with a KAT8 inhibitor, may be used; or a composition comprising a KAT8 inhibitor, wherein the KAT8 inhibitor is used in combination with a HAT1 inhibitor, may be used._

SEQUENCE LISTING

<110>  NATIONAL UNIVERSITY CORPORATION TOTTORI UNIVERSITY

<120>  Inhibitor of methylation-related enzyme HAT1 and KAT8

<130>  3782TTR

<150>  JP2015-215267
<151>  2015-10-30

<160>  33

<170>  PatentIn version 3.5

<210>  1
<211>  1682
<212>  RNA
<213>  Homo sapiens

<400>  1
cugugcgguc acuuccggcc cgggagcgcg cggguugauu cguccuuccu cagccgcggg      60

ugaucguagc ucggaaaugg cgggauuugg ugcuauggag aaauuuuugg uagaauauaa     120

gagugcagug gagaagaaac uggcagagua caaauguaac accaacacag caauugaacu     180

aaaauuaguu cguuuuccug aagaucuuga aaaugacauu agaacuuucu uuccugagua     240

uacccaucaa cucuuugggg augaugaaac ugcuuuuggu uacaaggguc uaaagauccu     300

guuauacuau auugcuggua gccugucaac aauguuccgu guugaauaug caucuaaagu     360

ugaugagaac uuugacugug uagaggcaga ugauguugag ggcaaaauua gacaaaucau     420

uccaccugga uuuugcacaa acacgaauga uuuccuuucu uuacuggaaa aggaaguuga     480

uuucaagcca uucggaaccu uacuucauac cuacucaguu cucaguccaa caggaggaga     540

aaacuuuacc uuucagauau auaaggcuga caugacaugu agaggcuuuc gagaauauca     600

ugaaaggcuu cagaccuuuu ugaugugguu uauugaaacu gcuagcuuua uugacgugga     660

ugaugaaaga uggcacuacu uucuaguauu ugagaaguau aauaaggaug gagcuacgcu     720

cuuugcgacc guaggcuaca ugacagucua uaauuacuau guguacccag acaaaacccg     780

gccacgugua agucagaugc ugauuuugac uccauuucaa ggucaaggcc auggugcuca     840

acuucuugaa acaguucaua gauacuacac ugaauuuccu acaguucuug auauuacagc     900

ggaagaucca uccaaaagcu augugaaauu acgagacuuu gugcuuguga agcuuuguca     960

agauuugccc uguuuuuccc gggaaaaauu aaugcaagga uucaaugaag auauggcgau    1020

agaggcacaa cagaaguuca aaauaaauaa gcaacacgcu agaaggguuu augaaauucu    1080

ucgacuacug guaacugaca ugagugaugc cgaacaauac agaagcuaca gacuggauau    1140

uaaaagaaga cuaauuagcc cauauaagaa aaagcagaga gaucuugcua agaugagaaa    1200

augucucaga ccagaagaac ugacaaacca gaugaaccaa auagaaauaa gcaugcaaca    1260

ugaacagcug gaagagaguu uucaggaacu aguggaagau uaccggcgug uuauugaacg 1320

acuugcucaa gaguaaagau uauacugcuc uguacaggaa gcuugcaaau uuucuguaca 1380

augugcugug aaaaaucuga ugacuuuaau uuuaaaaucu ugugacauuu ugcuuauacu 1440

aaaaguuauc uaucuuuagu ugaauauuuu cuuuuggaga gauuguauau uuuaaaauac 1500

uguuuagagu uuaugagcau auauugcauu uaaagaaaga uaaagcuucu gaaauacuac 1560

ugcaauugcu ucccuucuua aacaguauaa uaaaugcuua guugugauau guuaaugugu 1620

gaugauauga uucuuaaaua cuuacaauaa accucauucu uaaauacuua aaaaaaaaaa 1680

aa 1682

<210> 2
<211> 1596
<212> RNA
<213> Homo sapiens

<400> 2
   gagcgcgcgg guugauucgu ccuuccucag ccgcggguga ucguagcucg gaaauggcgg 60
   gauuuggugc uauggagaaa uuuuugguag aauauaagag ugcaguggag aagaaacugg 120
   cagaguacaa auguaacacc aacacagcaa uugaacuaaa auuagugaaa cugcuuuugg 180
   uuacaagggu cuaaagaucc uguuauacua uauugcuggu agccugucaa caauguuccg 240
   uguugaauau gcaucuaaag uugaugagaa cuuugacugu guagaggcag augauguuga 300
   gggcaaaauu agacaaauca uuccaccugg auuuugcaca aacacgaaug auuuccuuuc 360
   uuuacuggaa aaggaaguug auuucaagcc auucggaacc uuacuucaua ccuacucagu 420
   ucucagucca acaggaggag aaaacuuuac cuuucagaua uauaaggcug acaugacaug 480
   uagaggcuuu cgagaauauc augaaaggcu ucagaccuuu uugauguggu uuauugaaac 540
   ugcuagcuuu auugacgugg augaugaaag auggcacuac uuucuaguau uugagaagua 600
   uaauaaggau ggagcuacgc ucuuugcgac cguaggcuac augacagucu auaauuacua 660
   uguguaccca gacaaaaccc ggccacgugu aagucagaug cugauuuuga cuccauuuca 720
   aggucaaggc cauggugcuc aacuucuuga aacaguucau agauacuaca cugaauuucc 780
   uacaguucuu gauauuacag cggaagaucc auccaaaagc uaugugaaau uacgagacuu 840
   ugugcuugug aagcuuuguc aagauuugcc cuguuuuucc cgggaaaaau uaaugcaagg 900
   auucaaugaa gauauggcga uagaggcaca acagaaguuc aaaauaaaua agcaacacgc 960
   uagaaggguu uaugaaauuc uucgacuacu gguaacugac augagugaug ccgaacaaua 1020
   cagaagcuac agacuggaua uuaaaagaag acuaauuagc ccauauaaga aaaagcagag 1080
   agaucuugcu aagaugagaa aaugucucag accagaagaa cugacaaacc agaugaacca 1140
   aauagaaaua agcaugcaac augaacagcu ggaagagagu uuucaggaac uaguggaaga 1200
   uuaccggcgu guuauugaac gacuugcuca agaguaaaga uuauacugcu cuguacagga 1260
   agcuugcaaa uuuucuguac aaugugcugu gaaaaaucug augacuuuaa uuuuaaaauc 1320
   uugugacauu uugcuuauac uaaaaguuau cuaucuuuag uugaauauuu ucuuuuggag 1380
   agauuguaua uuuuaaaaua cuguuuagag uuuaugagca uauauugcau uuaaagaaag 1440
   auaaagcuuc ugaaauacua cugcaauugc uucccuucuu aaacaguaua auaaaugcuu 1500
   aguugugaua uguuaaugug ugaugauaug auucuuaaau acuuacaaua aaccucauuc 1560
   uuaaauacuu aaaaaaaaaa aaaaaaaaaa aaaaaa 1596

<210> 3
<211> 2420
<212> RNA
<213> Homo sapiens

<400> 3
uucaagucuu ucccauuuuc ugugugugug uuuuaacccc uuuuugaagu uuauagcaca 60

ucauuguacc ccuuuuuauu uuuugcuuau ggcuuuuuuc ugucugaauu uuuacugauu 120

uuuuuuuuuu uuggagacug agucucgcuc ugucaccagg cuggagugca guggcgcgau 180

cucggcaacc uuugccuucu ggauucaagc aauucugccu cagccuccgg aguagcuagg 240

acuacaguca cgcgccacca cguccagcua auuuuuguau uuuuagugga gaugggguuu 300

caccauguug gccaggauga uauccgucuc gaccucgcga ucccccugcc ucggccuccc 360

aaagugcuga aauuacaggc gugagccacc gcgcccagcc cauuuuuacu aaucauaugc 420

ccauuuuguc aggagauuug uuaucuugaa aguuucucuu gauagugaua ucuuccaaug 480

uuauuuauua uguauauuau ugaguagcca cuauguagua aaacuauugu gccaguagcu 540

cacaguuuau gacuuuaggc auacaugauu uuuaaauauu uaauacagug gaaagauguu 600

uucuuugugc cccuguuccu cuuccuucuu ucuuuaccca ugguaacuau gguuaccaga 660

uauuugugua uucuucuuga gacauuccau gcauuaucau uuccuuaucu gaagcacucu 720

gguagacuuu uuccaauacu uaaaaugaaa uucugucaua auuaucagag gacauucuca 780

uuuugcuuuu uacaucuuau aaugaaaaag acgacuuagg uauaaauccu agcaggauuu 840

ggugcuaugg agaaauuuuu gguagaauau aagagugcag uggagaagaa acuggcagag 900

uacaaaugua acaccaacac agcaauugaa cuaaaauuag uucguuuucc ugaagaucuu 960

gaaaaugaca uuagaacuuu cuuuccugag uauacccauc aacucuuugg ggaugaugaa 1020

acugcuuuug guuacaaggg ucuaaagauc cuguuauacu auauugcugg uagccuguca 1080

acaauguucc guguugaaua ugcaucuaaa guugaugaga acuuugacug uguagaggca 1140

gaugauguug agggcaaaau uagacaaauc auuccaccug gauuuugcac aaacacgaau 1200

gauuuccuuu cuuuacugga aaaggaaguu gauuucaagc cauucggaac cuuacuucau 1260

accuacucag uucucagucc aacaggagga gaaaacuuua ccuuucagau auauaaggcu 1320

gacaugacau guagaggcuu ucgagaauau caugaaaggc uucagaccuu uuugaugugg 1380

uuuauugaaa cugcuagcuu uauugacgug gaugaugaaa gauggcacua cuuucuagua 1440

uuugagaagu auaauaagga uggagcuacg cucuuugcga ccguaggcua caugacaguc 1500

uauaauuacu auguguaccc agacaaaacc cggccacgug uaagucagau gcugauuuug 1560

acuccauuuc aaggucaagg ccauggugcu caacuucuug aaacaguuca uagauacuac 1620

acugaauuuc cuacaguucu ugauauuaca gcggaagauc cauccaaaag cuaugugaaa 1680

uuacgagacu uugugcuugu gaagcuuugu caagauuugc ccuguuuuuc ccgggaaaaa 1740

uuaaugcaag gauucaauga agauauggug auagaggcac aacagaaguu caaaauaaau 1800

aagcaacacg cuagaagggu uuaugaaauu cuucgacuac ugguaacuga caugagugau 1860

gccgaacaau acagaagcua cagacuggau auuaaaagaa gacuaauuag cccauauaag 1920

aaaaagcaga gagaucuugc uaagaugaga aaaugucuca gaccagaaga acugacaaac 1980

cagaugaacc aaauagaaau aagcaugcaa caugaacagc uggaagagag uuuucaggaa 2040

cuaguggaag auuaccggcg uguuauugaa cgacuugcuc aagaguaaag auuauacugc 2100

ucuguacagg aagcuugcaa auuuucugua caaugugcug ugaaaaaucu gaugacuuua 2160

auuuuaaaau cuugugacau uuugcuuaua cuaaaaguua ucuaucuuua guugaauauu 2220

uucuuuugga gagauuguau auuuuaaaau acuguuuaga guuuaugagc auauauugca 2280

uuuaaagaaa gauaaagcuu cugaaauacu acugcaauug cuucccuucu uaaacaguau 2340

aauaaaugcu uaguugugau auguuaaugu gugaugauau gauucuuaaa uacuuacaau 2400

aaaccucauu cuuaaauacu 2420

<210> 4
<211> 1864
<212> RNA
<213> Homo sapiens

<400> 4
cugugcgguc acuuccggcc cgggagcgcg cggguugauu cguccuuccu cagccgcggg 60

ugaucguagc ucggaaaugg cgggucuccg ucuaccuucc acuacaguca acuucugagc 120

uagcgcauug cugccuugcc cugcucuggc gcucccacuu ggaucucgac ugacuucgua 180

gucgucucgu uaaucuguca cacuuuagua uuccgaggau cguauuuaga ugcccuuuga 240

gaaagacuuu guuccugaac ugcucccuuc ucuuuuaggg auuuggugcu auggagaaau 300

uuuugguaga auauaagagu gcaguggaga agaaacuggc agaguacaaa uguaacacca 360

acacagcaau ugaacuaaaa uuaguucguu uuccugaaga ucuugaaaau gacauuagaa 420

cuuucuuucc ugaguauacc caucaacucu uuggggauga ugaaacugcu uuugguuaca 480

agggucuaaa gauccuguua uacuauauug cugguagccu gucaacaaug uuccguguug 540

aauaugcauc uaaaguugau gagaacuuug acuguguaga ggcagaugau guugagggca 600

aaauuagaca aaucauucca ccuggauuuu gcacaaacac gaaugauuuc cuuucuuuac 660

uggaaaagga aguugauuuc aagccauucg gaaccuuacu ucauaccuac ucaguucuca 720

guccaacagg aggagaaaac uuuaccuuuc agauauauaa ggcugacaug acauguagag 780

gcuuucgaga auaucaugaa aggcuucaga ccuuuuugau gugguuuauu gaaacugcua 840

gcuuuauuga cguggaugau gaaagauggc acuacuuucu aguauuugag aaguauaaua 900

aggauggagc uacgcucuuu gcgaccguag gcuacaugac agucuauaau uacuaugugu 960

acccagacaa aacccggcca cguguaaguc agaugcugau uuugacucca uuucaagguc 1020

aaggccaugg ugcucaacuu cuugaaacag uucauagaua cuacacugaa uuuccuacag 1080

uucuugauau uacagcggaa gauccaucca aaagcuaugu gaaauuacga gacuuugugc 1140

uugugaagcu uugucaagau uugcccuguu uuucccggga aaaauuaaug caaggauuca 1200

augaagauau ggugauagag gcacaacaga aguucaaaau aaauaagcaa cacgcuagaa 1260

ggguuuauga aauucuucga cuacugguaa cugacaugag ugaugccgaa caauacagaa 1320

gcuacagacu ggauauuaaa agaagacuaa uuagcccaua uaagaaaaag cagagagauc 1380

uugcuaagau gagaaaaugu cucagaccag aagaacugac aaaccagaug aaccaaauag 1440

aaauaagcau gcaacaugaa cagcuggaag agaguuuuca ggaacuagug gaagauuacc 1500

ggcguguuau ugaacgacuu gcucaagagu aaagauuaua cugcucugua caggaagcuu 1560

gcaaauuuuc uguacaaugu gcugugaaaa aucugaugac uuuaauuuua aaaucuugug 1620

acauuuugcu uauacuaaaa guuaucuauc uuuaguugaa uauuuucuuu uggagagauu 1680

guauauuuua aaauacuguu uagaguuuau gagcauauau ugcauuuaaa gaaagauaaa 1740

gcuucugaaa uacuacugca auugcuuccc uucuuaaaca guauaauaaa ugcuuaguug 1800

ugauauguua augugugaug auaugauucu uaaauacuua caauaaaccu cauucuuaaa 1860

uacu 1864

<210> 5
<211> 1546
<212> RNA
<213> Homo sapiens

<400> 5
gucacuuccc uucccgcgau ggcggcacag ggagcugcug cggcgguugc ggcggggacu 60

ucaggggucg cgggggaggg cgagcccggg cccggggaga augcggccgc ugaggggacc 120

gccccauccc cgggccgcgu cucuccgccg accccggcgc gcggcgagcc ggaagucacg 180

guggagaucg gagaaacgua ccugugccgg cgaccggaua gcaccuggca uucugcugaa 240

gugauccagu cucgagugaa cgaccaggag ggccgagagg aauucuaugu acacuacgug 300

ggcuuuaacc ggcggcugga cgagugggua gacaagaacc ggcuggcgcu gaccaagaca 360

gugaaggaug cuguacagaa gaacucagag aaguaccuga gcgagcucgc agagcagccu 420

gagcgcaaga ucacucgcaa ccaaaagcgc aagcaugaug agaucaacca ugugcagaag 480

acuuaugcag agauggaccc caccacagca gccuuggaga aggagcauga ggcgaucacc 540

aaggugaagu auguggacaa gauccacauc gggaacuacg aaauugaugc cugguauuuc 600

ucaccauucc ccgaagacua ugggaaacag cccaagcucu ggcucugcga guacugccuc 660

aaguacauga aauaugagaa gagcuaccgc uuccacuugg gucagugcca guggcggcag 720

ccccccggga aagagaucua ccgcaagagc aacaucuccg uguacgaagu ugauggcaaa 780

gaccauaaga uuuacuguca gaaccugugu cugcuggcca agcuuuuccu ggaccauaag 840

acacuguacu uugacgugga gccguucguc uuuuacaucc ugacugaggu ggaccggcag 900

ggggcccaca uuguuggcua cuucuccaag gagaaggagu ccccggaugg aaacaaugug 960

gccugcaucc ugaccuugcc ccccuaccaa cgccgcggcu acgggaaguu ccucaucgcu 1020

uucaguuaug agcucuccaa gcuggagagc acagucggcu ccccggagaa gccacugucu 1080

gaccugggca agcucagcua ccgcagcuac ugguccuggg ugcugcuaga gauccugcgg 1140

gacuuccggg gcacacuguc caucaaggac cucagccaga ugaccaguau cacccaaaau 1200

gacaucauca guacccugca aucccucaau auggucaagu acuggaaggg ccagcacgug 1260

aucuguguca cacccaagcu gguggaggag caccucaaaa gugcccagua uaagaaacca 1320

cccaucacag uggacuccgu cugccucaag ugggcacccc ccaagcacaa gcaagucaag 1380

cucuccaaga agugagcagc cuggccccug cugucggacc ugagccuccu ggcucccagc 1440

cuguaaauau guauagaccu guuuugucau uuuuuuaaua aagucaguuc ugguggcccu 1500

ggacuuugga ggggaagggg aggccaagaa aaaaaaaaaa aaaaaa 1546

<210> 6
<211> 1840
<212> RNA
<213> Homo sapiens

<400> 6
   gucacuuccc uucccgcgau ggcggcacag ggagcugcug cggcgguugc ggcggggacu 60
   ucaggggucg cgggggaggg cgagcccggg cccggggaga augcggccgc ugaggggacc 120
   gccccauccc cgggccgcgu cucuccgccg accccggcgc gcggcgagcc ggaagucacg 180
   guggagaucg gagaaacgua ccugugccgg cgaccggaua gcaccuggca uucugcugaa 240
   gugauccagu cucgagugaa cgaccaggag ggccgagagg aauucuaugu acacuacgug 300
   ggcuuuaacc ggcggcugga cgagugggua gacaagaacc ggcuggcgcu gaccaagaca 360
   gugaaggaug cuguacagaa gaacucagag aaguaccuga gcgagcucgc agagcagccu 420
   gagcgcaaga ucacucgcaa ccaaaagcgc aagcaugaug agaucaacca ugugcagaag 480
   acuuaugcag agauggaccc caccacagca gccuuggaga aggagcauga ggcgaucacc 540
   aaggugaagu auguggacaa gauccacauc gggaacuacg aaauugaugc cugguauuuc 600
   ucaccauucc ccgaagacua ugggaaacag cccaagcucu ggcucugcga guacugccuc 660
   aaguacauga aauaugagaa gagcuaccgc uuccacuugg gucagugcca guggcggcag 720
   cccccgggga aagagaucua ccgcaagagc aacaucuccg uguacgaagu ugauggcaaa 780
   gaccauaaga uuuacuguca gaaccugugu cugcuggcca agcuuuuccu ggaccauaag 840
   acacuguacu uugacgugga gccguucguc uuuuacaucc ugacugaggu ggaccggcag 900
   ggggcccaca uuguuggcua cuucuccaag gagaaggagu ccccggaugg aaacaaugug 960
   gccugcaucc ugaccuugcc ccccuaccaa cgccgcggcu acgggaaguu ccucaucgcu 1020
   uucaguuaug agcucuccaa gcuggagagc acagucggcu ccccggagaa gccacugucu 1080
   gaccugggca agcucagcua ccgcagcuac ugguccuggg ugcugcuaga gauccugcgg 1140
   gacuuccggg gcacacuguc caucaaggac cucagccaga ugaccaguau cacccaaaau 1200
   gacaucauca guacccugca aucccucaau auggucaagu acuggaaggg ccagcacgug 1260
   aucuguguca cacccaagcu gguggaggag caccucaaaa gugcccagua uaagaaacca 1320
   cccaucacag gugggugggg ggcugcugug ugucgggggc ggugggggag ugucaguaua 1380
   uggacuggua ggagucaagg ccuccuuauu gcugucacau gaucccaaac cagucagacc 1440
   agcucccagg auggagcccg gggcaggccu cucauuccug ggcuucccug ccccucccca 1500
   gcacagccug cccuuuuguu cucaccugga ggaggugaaa cuggccugag cccagaggag 1560
   ggcagccagg ugugagcugg ccuggcccag gcccagcccu gccucccgcc ccuucucccc 1620
   acaguggacu ccgucugccu caaguggcca ccccccaagc acaagcaagu caagcucucc 1680
   aagaagugag cagccuggcc ccugcugucg gaccugagcc uccuggcucc cagccuguaa 1740
   auauguauag accuguuuug ucauuuuuuu aauaaaguca guucuggugg cccuggacuu 1800
   uggaggggaa ggggaggcca agaaaaaaaa aaaaaaaaaa 1840

<210> 7
<211> 2197
<212> RNA
<213> Homo sapiens

<400> 7
gacaggcugu cagugaggcc aagauccggg ggcugggagu ggagaggagg aggggcgggg 60

cuugaggaaa gaacgccgcg uuccgggcgc ugagaaacca gccggguugu gggaggcugu 120

ugaccuugaa uuaugccgag cgacgccuac aaacccaccg cucaggccuu caccaggauu 180

guucccauuc cacuuccuug cccagucuua ggcuucauuc cuuuuucuug cuaacgcugc 240

uuccucaccc ucucuugucu cugcgucuuc uuuuuccauu ugucccuggc agccauccgc 300

agagagaaga ccuuccagaa acaacaggcu ucccuuuccu aaaguucugc ugccuucucu 360

cauuuucaaa auuaacccca aacuccuuag gguggcauuc auuuuuguga ccucuccagu 420

uucuagccaa cacuaggaaa gggcauugcc aggccagaac acacugugcc cucugaagac 480

cacacgcccu uuaccaccug ugcccuuugc uuggaaugcu uuuucuuccc uuucuccuug 540

uuugccugcc uagcuccuac ucauccucuu agcuucauau ccuuugugau gucauccuug 600

auuccccuuc aggcaaagug agugguuccc uucucuaugu uccugcaaca uuuuuuuccu 660

accucaguca uaguuuuugu aacauuaugu uguaauuuuc ucucucuguc uuccuccauc 720

auacugggaa cuucuggagg gcagcacuuc uugugauuca ucacuguguc cucuguaccc 780

ggcaacaaca cagcauaggg ccagacacgu agugguugcc uuacucauuu auugaaugcu 840

cugcuaauug ucaggugcgc ugcuaaaaau uuuggaggca uuaauucacc uucuaagauu 900

cugcugaagu gauccagucu cgagugaacg accaggaggg ccgagaggaa uucuauguac 960

acuacguggg cuuuaaccgg cggcuggacg aguggguaga caagaaccgg cuggcgcuga 1020

ccaagacagu gaaggaugcu guacagaaga acucagagaa guaccugagc gagcucgcag 1080

agcagccuga gcgcaagauc acucgcaacc aaaagcgcaa gcaugaugag aucaaccaug 1140

ugcagaagac uuaugcagag auggacccca ccacagcagc cuuggagaag gagcaugagg 1200

cgaucaccaa ggugaaguau guggacaaga uccacaucgg gaacuacgaa auugaugccu 1260

gguauuucuc accauucccc gaagacuaug ggaaacagcc caagcucugg cucugcgagu 1320

acugccucaa guacaugaaa uaugagaaga gcuaccgcuu ccacuugggu cagugccagu 1380

ggcggcagcc ccccgggaaa gagaucuacc gcaagagcaa caucuccgug uacgaaguug 1440

auggcaaaga ccauaagauu uacugucaga accugugucu gcuggccaag cuuuuccugg 1500

accauaagac acuguacuuu gacguggagc cguucgucuu uuacauccug acugaggugg 1560

accggcaggg ggcccacauu guuggcuacu ucuccaagga gaaggagucc ccggauggaa 1620

acaauguggc cugcauccug accuugcccc ccuaccaacg ccgcggcuac gggaaguucc 1680

ucaucgcuuu caguuaugag cucuccaagc uggagagcac agucggcucc ccggagaagc 1740

cacugucuga ccugggcaag cucagcuacc gcagcuacug guccugggug cugcuagaga 1800

uccugcggga cuuccggggc acacugucca ucaaggaccu cagccagaug accaguauca 1860

cccaaaauga caucaucagu acccugcaau cccucaauau ggucaaguac uggaagggcc 1920

agcacgugau cugugucaca cccaagcugg uggaggagca ccucaaaagu gcccaguaua 1980

agaaaccacc caucacagug gacuccgucu gccucaagug ggcacccccc aagcacaagc 2040

aagucaagcu cuccaagaag ugagcagccu ggccccugcu gucggaccug agccuccugg 2100

cucccagccu guaaauaugu auagaccugu uuugucauuu uuuuaauaaa gucaguucug 2160

guggcccugg acuuuggagg ggaaggggag gccaaga 2197

<210> 8
<211> 2070
<212> RNA
<213> Homo sapiens

<400> 8
gacaggcugu cagugaggcc aagauccggg ggcugggagu ggagaggagg aggggcgggg 60

cuugaggaaa gaacgccgcg uuccgggcgc ugagaaacca gccggguugu gggaggcugu 120

ugaccuugaa uuaugccgag cgacgccuac aaacccaccg cucaggccuu caccaggauu 180

guucccauuc cacuuccuug cccagucuua ggcuucauuc cuuuuucuug cuaacgcugc 240

uuccucaccc ucucuugucu cugcgucuuc uuuuuccauu ugucccuggc agccauccgc 300

agagagaaga ccuuccagaa acaacaggcu ucccuuuccu aaaguucugc ugccuucucu 360

cauuuucaaa auuaacccca aacuccuuag gguggcauuc auuuuuguga ccucuccagu 420

uucuagccaa cacuaggaaa gggcauugcc aggccagaac acacugugcc cucugaagac 480

cacacgcccu uuaccaccug ugcccuuugc uuggaaugcu uuuucuuccc uuucuccuug 540

uuugccugcc uagcuccuac ucauccucuu agcuucauau ccuuugugau gucauccuug 600

auuccccuuc aggcaaacac uucuugugau ucaucacugu guccucugua cccggcaaca 660

acacagcaua gggccagaca cguagugguu gccuuacuca uuuauugaau gcucugcuaa 720

uugucaggug cgcugcuaaa aauuuuggag gcauuaauuc accuucuaag auucugcuga 780

agugauccag ucucgaguga acgaccagga gggccgagag gaauucuaug uacacuacgu 840

gggcuuuaac cggcggcugg acgagugggu agacaagaac cggcuggcgc ugaccaagac 900

agugaaggau gcuguacaga agaacucaga gaaguaccug agcgagcucg cagagcagcc 960

ugagcgcaag aucacucgca accaaaagcg caagcaugau gagaucaacc augugcagaa 1020

gacuuaugca gagauggacc ccaccacagc agccuuggag aaggagcaug aggcgaucac 1080

caaggugaag uauguggaca agauccacau cgggaacuac gaaauugaug ccugguauuu 1140

cucaccauuc cccgaagacu augggaaaca gcccaagcuc uggcucugcg aguacugccu 1200

caaguacaug aaauaugaga agagcuaccg cuuccacuug ggucagugcc aguggcggca 1260

gcccccgggg aaagagaucu accgcaagag caacaucucc guguacgaag uugauggcaa 1320

agaccauaag auuuacuguc agaaccugug ucugcuggcc aagcuuuucc uggaccauaa 1380

gacacuguac uuugacgugg agccguucgu cuuuuacauc cugacugagg uggaccggca 1440

gggggcccac auuguuggcu acuucuccaa ggagaaggag uccccggaug gaaacaaugu 1500

ggccugcauc cugaccuugc cccccuacca acgccgcggc uacgggaagu uccucaucgc 1560

uuucaguuau gagcucucca agcuggagag cacagucggc uccccggaga agccacuguc 1620

ugaccugggc aagcucagcu accgcagcua cugguccugg gugcugcuag agauccugcg 1680

ggacuuccgg ggcacacugu ccaucaagga ccucagccag augaccagua ucacccaaaa 1740

ugacaucauc aguacccugc aaucccucaa uauggucaag uacuggaagg gccagcacgu 1800

gaucugugu c acacccaagc ugguggagga gcaccucaaa agugcccagu auaagaaacc 1860

acccaucaca guggacuccg ucugccucaa gugggcaccc cccaagcaca agcaagucaa 1920

gcucuccaag aagugagcag ccuggccccu gcugucggac cugagccucc uggcucccag 1980

ccuguaaaua uguauagacc uguuuuguca uuuuuuuaau aaagucaguu cugguggccc 2040

uggacuuugg aggggaaggg gaggccaaga 2070

<210> 9
<211> 2075
<212> RNA
<213> Homo sapiens

<400> 9
gacaggcugu cagugaggcc aagauccggg ggcugggagu ggagaggagg aggggcgggg 60

cuugaggaaa gaacgccgcg uuccgggcgc ugagaaacca gccggguugu gggaggcugu 120

ugaccuugaa uuaugccgag cgacgccuac aaacccaccg cucaggccuu caccaggauu 180

guucccauuc cacuuccuug cccagucuua ggcuucauuc cuuuuucuug cuaacgcugc 240

uuccucaccc ucucuugucu cugcgucuuc uuuuuccauu ugucccuggc agccauccgc 300

agagagaaga ccuuccagaa acaacaggcu ucccuuuccu aaaguucugc ugccuucucu 360

cauuuucaaa auuaacccca aacuccuuag gguggcauuc auuuuuguga ccucuccagu 420

uucuagccaa cacuaggaaa gggcauugcc aggccagaac acacugugcc cucugaagac 480

cacacgcccu uuaccaccug ugcccuuugc uuggaaugcu uuuucuuccc uuucuccuug 540

uuugccugcc uagcuccuac ucauccucuu agcuucauau ccuuugugau gucauccuug 600

auuccccuuc aggcaaaggc agcacuucuu gugauucauc acuguguccu cuguacccgg 660

caacaacaca gcauagggcc agacacguag ugguugccuu acucauuuau ugaaugcucu 720

gcuaauuguc aggugcgcug cuaaaaauuu uggaggcauu aauucaccuu cuaagauucu 780

gcugaaguga uccagucucg agugaacgac caggagggcc gagaggaauu cuauguacac 840

uacgugggcu uuaaccggcg gcuggacgag uggguagaca agaaccggcu ggcgcugacc 900

aagacaguga aggaugcugu acagaagaac ucagagaagu accugagcga gcucgcagag 960

cagccugagc gcaagaucac ucgcaaccaa aagcgcaagc augaugagau caaccaugug 1020

cagaagacuu augcagagau ggaccccacc acagcagccu uggagaagga gcaugaggcg 1080

aucaccaagg ugaaguaugu ggacaagauc cacaucggga acuacgaaau ugaugccugg 1140

uauuucucac cauuccccga agacuauggg aaacagccca agcucuggcu cugcgaguac 1200

ugccucaagu acaugaaaua ugagaagagc uaccgcuucc acuuggguca gugccagugg 1260

cggcagcccc ccgggaaaga gaucuaccgc aagagcaaca ucuccgugua cgaaguugau 1320

ggcaaagacc auaagauuua cugucagaac cugugucugc uggccaagcu uuuccuggac 1380

cauaagacac uguacuuuga cguggagccg uucgucuuuu acauccugac ugagguggac 1440

cggcaggggg cccacauugu uggcuacuuc uccaaggaga aggagucccc ggauggaaac 1500

aauguggccu gcauccugac cuugcccccc uaccaacgcc gcggcuacgg gaaguuccuc 1560

aucgcuuuca guuaugagcu cuccaagcug gagagcacag ucggcucccc ggagaagcca 1620

cugucugacc ugggcaagcu cagcuaccgc agcuacuggu ccugggugcu gcuagagauc 1680

cugcgggacu uccggggcac acuguccauc aaggaccuca gccagaugac caguaucacc 1740

caaaaugaca ucaucaguac ccugcaaucc cucaauaugg ucaaguacug gaagggccag 1800

cacgugaucu gugucacacc caagcuggug gaggagcacc ucaaaagugc ccaguauaag 1860

aaaccaccca ucacagugga cuccgucugc cucaaguggg caccccccaa gcacaagcaa 1920

gucaagcucu ccaagaagug agcagccugg ccccugcugu cggaccugag ccuccuggcu 1980

cccagccugu aaauauguau agaccuguuu ugucauuuuu uuaauaaagu caguucuggu 2040

ggcccuggac uuuggagggg aaggggaggc caaga 2075

<210> 10
<211> 21
<212> RNA
<213> Homo sapiens

<400> 10
ccaacacagc aauugaacua a 21

<210> 11
<211> 21
<212> RNA
<213> Homo sapiens

<400> 11
gccauucgga accuuacuuc a 21

<210> 12
<211> 21
<212> RNA
<213> Homo sapiens

<400> 12
gcuuucgaga auaucaugaa a 21

<210> 13
<211> 21

<212>  RNA
<213>  Homo sapiens

<400>  13
gcuagaaggg uuuaugaaau u                                                21


<210>  14
<211>  21
<212>  RNA
<213>  Artificial Sequence

<220>
<223>  Guide strand

<400>  14
gguugugucg uuaacuugau u                                                21


<210>  15
<211>  21
<212>  RNA
<213>  Artificial Sequence

<220>
<223>  Guide strand

<400>  15
cgguaagccu uggaaugaag u                                                21


<210>  16
<211>  21
<212>  RNA
<213>  Artificial Sequence

<220>
<223>  Guide strand

<400>  16
cgaaagcucu uauaguacuu u                                                21


<210>  17
<211>  21
<212>  RNA
<213>  Artificial Sequence

<220>
<223>  Guide strand

<400>  17
cgaucuuccc aaauacuuua a                                                21


<210>  18
<211>  21
<212>  RNA
<213>  Artificial Sequence

<220>
<223>  Passenger strand

<400>  18
ccaacacagc aauugaacua a                                                21

<210>  19
<211>  21
<212>  RNA
<213>  Artificial Sequence

<220>
<223>  Passenger strand

<400>  19
gccauucgga accuuacuuc a                                                21

<210>  20
<211>  21
<212>  RNA
<213>  Artificial Sequence

<220>
<223>  Passenger strand

<400>  20
gcuuucgaga auaucaugaa a                                                21

<210>  21
<211>  21
<212>  RNA
<213>  Artificial Sequence

<220>
<223>  Passenger strand

<400>  21
gcuagaaggg uuuaugaaau u                                                21

<210>  22
<211>  21
<212>  RNA
<213>  Homo sapiens

<400>  22
ccgagaggaa uucuauguac a                                                21

<210>  23
<211>  21
<212>  RNA
<213>  Homo sapiens

<400>  23
gggaacuacg aaauugaugc c                                                21

<210>  24
<211>  21
<212>  RNA
<213>  Homo sapiens

<400>  24
caccaaggug aaguaugugg a                                                21


<210>  25
<211>  21
<212>  RNA
<213>  Homo sapiens

<400>  25
guacugccuc aaguacauga a                                                21


<210>  26
<211>  21
<212>  RNA
<213>  Artificial Sequence

<220>
<223>  Guide strand

<400>  26
ggcucuccuu aagauacaug u                                                21


<210>  27
<211>  21
<212>  RNA
<213>  Artificial Sequence

<220>
<223>  Guide strand

<400>  27
cccuugaugc uuuaacuacg g                                                21


<210>  28
<211>  21
<212>  RNA
<213>  Artificial Sequence

<220>
<223>  Guide strand

<400>  28
gugguuccac uucauacacc u                                                21


<210>  29
<211>  21
<212>  RNA
<213>  Artificial Sequence

<220>
<223>  Guide strand

<400>  29
caugacggag uucauguacu u                                                21


<210>  30
<211>  21

<212>  RNA
<213>  Artificial Sequence

<220>
<223>  Passenger strand

<400>  30
ccgagaggaa uucuauguac a                                                21


<210>  31
<211>  21
<212>  RNA
<213>  Artificial Sequence

<220>
<223>  Passenger strand

<400>  31
gggaacuacg aaauugaugc c                                                21


<210>  32
<211>  21
<212>  RNA
<213>  Artificial Sequence

<220>
<223>  Passenger strand

<400>  32
caccaaggug aaguaugugg a                                                21


<210>  33
<211>  21
<212>  RNA
<213>  Artificial Sequence

<220>
<223>  Passenger strand

<400>  33
guacugccuc aaguacauga a                                                21

## Description

### Technical Field

The present invention relates to therapeutic drugs for malignant tumors, methods for producing a stem cell, DNA damage-ameliorating agents, or HAT1 and KAT8 inhibitors.

### Background Art

Examples of the leading causes of human death include malignant tumors, heart disease, and cerebrovascular disease. Among them, the mechanism of causing malignant tumors is complicated, so that the malignant tumors, in particular, can be said to be hard-to-treat diseases. Nowadays, nivolumab or palbociclib should serve as an anti-neoplastic agent with a novel mechanism of action (Non-Patent Literature 1). The present inventor has reported, in Non-Patent Literature 1, that hTERT mRNA is applicable as a malignant tumor biomarker. The present inventor has also reported, in Patent Literatures 1, 2, and 3, that specific RNA strands (e.g., miR-47 siRNA, has-mir-520d, ELAVL2 siRNA) may be used for treatment of malignant tumors.

Meanwhile, iPS cells have recently received attention in pharmaceutical industries. In 2012, an iPS cell researcher was awarded a Nobel Prize in Physiology and Medicine. In 2014, the first iPS cell implantation was conducted on a patient with age-related macular degeneration. Regarding the iPS cell-related technologies, Patent Literature 4, for example, describes that introduction of 4 genes (Oct3/4, Klf4, Sox2, and c-Myc) into a cell causes the cell to be reprogrammed into an iPS cell. Patent Literature 5 also describes that introduction of 3 genes (Oct3/4, Klf4, and Sox2) and one miRNA (e.g., hsa-miR-372) into a cell leads to production of an iPS cell. In addition, the present inventor has reported, in Patent Literatures 1, 2, and 3, that introduction of specific RNA strands into a cell leads to production of an iPS cell.

### Citation List

### Patent Literature

[Patent Literature 1] WO2012/008301
[Patent Literature 2] WO2012/008302
[Patent Literature 3] WO2014/097875
[Patent Literature 4] WO2007/069666
[Patent Literature 5] WO2009/075119

### Non Patent Literature

[Non Patent Literature 1] "A Review of 2014 Cancer Drug Approvals, With a Look at 2015 and Beyond" Butler et al., P T. 2015 Mar; 40(3):191-205.
[Non Patent Literature 2] "A novel biomarker TERT mRNA is applicable for early detection of hepatoma." Miura et al., BMC Gastroenterol. 2010 May 18; 10:46.

### Summary of Invention

### Technical Problem

Nevertheless, the malignant tumors are still one of the current leading causes of human death. Hence, conventional treatment strategies are simply insufficient. Meanwhile, development of iPS cell production technology and clinical application is in progress, so that the production procedures have limitations.

The present invention has been made in view of the above situations. The purpose of the present invention is to provide novel therapeutic drugs for malignant tumors, methods for producing a stem cell, or DNA damage-ameliorating agents, etc.

### Solution to Problem

As described in below-described Examples, the present inventor discovered that when shRNAs for inhibiting expression of HAT1 and KAT8 were introduced into a malignant tumor cell, the malignant tumor cell expressed sternness characteristics. Further, when malignant tumor cells were treated with a low-molecular-weight compound that inhibits HAT1 and KAT8, the growth of the malignant tumor cells was markedly suppressed. Furthermore, when malignant tumor cells that were subject to lethal DNA damage caused by UV irradiation were treated with the above low-molecular-weight compound, the DNA damage was markedly ameliorated. Then, the present inventor has completed the present invention based on the results.

Specifically, an aspect of the present invention provides a therapeutic drug for a malignant tumor, comprising a HAT1 and KAT8 inhibitor.

In addition, another aspect of the present invention provides a kit for treating a malignant tumor, comprising a HAT1 inhibitor and a KAT8 inhibitor.

In addition, another aspect of the present invention provides a therapeutic drug for a malignant tumor, comprising a HAT1 inhibitor, wherein the HAT1 inhibitor is used in combination with a KAT8 inhibitor.

In addition, another aspect of the present invention provides a therapeutic drug for a malignant tumor, comprising a KAT8 inhibitor, wherein the KAT8 inhibitor is used in combination with a HAT1 inhibitor.

In addition, another aspect of the present invention provides a method for producing a stem cell, comprising the step of inhibiting HAT1 and KAT8.

In addition, another aspect of the present invention provides a sternness characteristic inducer comprising a HAT1 and KAT8 inhibitor.

In addition, another aspect of the present invention provides a sternness characteristic-inducing kit comprising a HAT1 inhibitor and a KAT8 inhibitor.

In addition, another aspect of the present invention provides a sternness characteristic inducer comprising a HAT1 inhibitor, wherein the HAT1 inhibitor is used in combination with a KAT8 inhibitor.

In addition, another aspect of the present invention provides a sternness characteristic inducer comprising a KAT8 inhibitor, wherein the KAT8 inhibitor is used in combination with a HAT1 inhibitor.

In addition, another aspect of the present invention provides a DNA damage-ameliorating agent comprising a HAT1 and KAT8 inhibitor.

In addition, another aspect of the present invention provides a DNA damage-ameliorating kit comprising a HAT1 inhibitor and a KAT8 inhibitor.

In addition, another aspect of the present invention provides a DNA damage-ameliorating agent comprising a HAT1 inhibitor, wherein the HAT1 inhibitor is used in combination with a KAT8 inhibitor.

In addition, another aspect of the present invention provides a DNA damage-ameliorating agent comprising a KAT8 inhibitor, wherein the KAT8 inhibitor is used in combination with a HAT1 inhibitor.

### Advantageous Effects of Invention

The present invention makes it possible to, for example, treat a malignant tumor, produce a stem cell, ameliorate DNA damage, or inhibit HAT1 and KAT8.

### Brief Description of the Drawings

[FIG. 1] FIG. 1 is pictures illustrating the results of examining the cell morphology of HLF cells after introduction of shHAT1/shKAT8.
[FIG. 2] FIG. 2 is graphs and pictures illustrating the results of examining the cell shape of HLF cells after introduction of shHAT1/shKAT8.
[FIG. 3] FIG. 3 is pictures illustrating the results of analyzing stem cell markers expressed on HLF cells after introduction of shHAT1/shKAT8.
[FIG. 4] FIG. 4 is pictures illustrating the results of examining how HLF cells after introduction of shHAT1/shKAT8 were transformed and differentiated.
[FIG. 5] FIG. 5 is tables showing the CAS registry number and the HAT1- and KAT8-binding strengths of each compound such as chlorpropamide.
[FIG. 6] FIG. 6 is graphs showing the results of examining an inhibitory effect of chlorpropamide, etc., on expression of HAT1 or KAT8.
[FIG. 7] FIG. 7 is tables showing the CAS registry number and the HAT1- or KAT8-binding strength of each compound such as fosinopril.
[FIGS. 8 to 10] FIGS. 8 to 10 are pictures illustrating the results of examining a growth suppression effect of oxprenolol hydrochloride, etc., on malignant tumor cells.
[FIG. 11] FIG. 11 is a picture illustrating the results of examining a growth suppression effect of chlorpropamide on malignant tumor cells.
[FIG. 12] FIG. 12 is pictures illustrating the results of examining how rebamipide affected malignant tumor cells.
[FIG. 13] FIG. 13 is a table showing the results of examining an anti-tumor effect of chlorpropamide on various tumors.

### Description of Embodiments

Hereinafter, embodiments of the present invention will be described in detail. Note that descriptions are not repeated so as to avoid redundancy.

An embodiment of the present invention provides a therapeutic drug for a malignant tumor, comprising a HAT1 and KAT8 inhibitor. This therapeutic drug may be used to treat malignant tumors. In below-described Examples, a malignant tumor was transformed into a stem cell after introduction of shRNAs against HAT1 and KAT8 into a cell. That is, the inhibition of HAT1 and KAT8 resulted in induction of the malignant tumor into the stem cell. In this way, an anti-malignant tumor effect was elicited. Further, after malignant tumor cells were treated with a low-molecular-weight compound that inhibits HAT1 and KAT8, the growth of the malignant tumor cells was suppressed.

As used herein, the term "HAT1" relates to a concept including the HAT1 gene and protein. Detailed information on the HAT1 gene and protein can be seen in Web sites of HGNC (HUGO Gene Nomenclature Committee) or UniProt (Universal Protein Resource), etc. The HGNC ID of the HAT1 gene as described in HGNC site is HGNC: 4821, and its chromosomal location is 2Q31.2-q33.1. Meanwhile, HAT1 is sometimes called KAT1. Those referring to substantially the same ones as HAT1 can be included in the HAT1. The HAT1 protein encompasses HAT1 gene-derived proteins and proteins expressed from the HAT1 gene. The general name of the HAT1 protein has no limitation. In the UniProt, the HAT1 protein is called Histone acetyltransferase type B catalytic subunit, which is included in the HAT1 protein. The HAT1 mRNA may contain, for example, the nucleotide sequence set forth in SEQ ID NO: 1. The HAT1 mRNA may also have isoforms, which may contain, for example, the nucleotide sequence set forth in SEQ ID NO: 2, 3, or 4.

As used herein, the term "KAT8" relates to a concept including the KAT8 gene and protein. Detailed information on the KAT8 gene and protein can be seen in Web sites of HGNC (HUGO Gene Nomenclature Committee) or UniProt (Universal Protein Resource), etc. The HGNC ID of the KAT8 gene as described in HGNC site is HGNC: 17933, and its chromosomal location is 16p11.1. Meanwhile, KAT8 is sometimes called MYST1, FLJ14040, hMOF, MOF, or ZC2HC8. Those referring to substantially the same ones as KAT8 can be included in the KAT8. The KAT8 protein encompasses KAT8 gene-derived proteins and proteins expressed from the KAT8 gene. The general name of the KAT8 protein has no limitation. In the UniProt, the KAT8 protein is called Histone acetyltransferase KAT8, which is included in the KAT8 protein. The KAT8 mRNA may contain, for example, the nucleotide sequence set forth in SEQ ID NO: 5. The HAT1 mRNA may also have isoforms, which may contain, for example, the nucleotide sequence set forth in SEQ ID NO: 6, 7, or 8.

As used herein, the "HAT1 and KAT8 inhibitor" is an inhibitor for inhibiting both HAT1 and KAT8. This HAT1 and KAT8 inhibitor may be a composition comprising a HAT1 inhibitor and a KAT8 inhibitor. The HAT1 and KAT8 inhibitor may include a composition for inhibiting gene expression of both HAT1 and KAT8 or for inhibiting protein function of both HAT1 and KAT. The HAT1 and KAT8 inhibitor may comprise one active ingredient (e.g., an ingredient that inhibits both HAT1 and KAT8) or two active ingredients (e.g., an ingredient that inhibits HAT1 and an ingredient that inhibits KAT8).

As used herein, examples of the "malignant tumor" include tumors caused by a mutation in a normal cell. The malignant tumors may occur in any organs and tissues in the body. Each malignant tumor may be, for example, at least one selected from the group consisting of lung cancer, esophagus cancer, gastric cancer, liver cancer, pancreatic cancer, renal cancer, adrenal cancer, biliary tract cancer, breast cancer, colon cancer, small intestinal cancer, ovarian cancer, uterine cancer, bladder cancer, prostate cancer, ureteral cancer, renal pelvis cancer, ureteral cancer, penile cancer, testicular cancer, brain tumor, cancer in central nervous system, cancer in peripheral nervous system, head and neck carcinoma, glioma, glioblastoma multiforme, skin cancer, melanoma, thyroid cancer, salivary gland cancer, malignant lymphoma, carcinoma, sarcoma, and hematological malignancies. The above liver cancer may be, for example, an epithelial tumor or nonepithelial tumor, and may be hepatocyte carcinoma or cholangiocellular carcinoma.

An embodiment of the present invention provides a kit for treating a malignant tumor, comprising a HAT1 inhibitor and a KAT8 inhibitor. This kit may be used to treat malignant tumors. This kit may further contain, for example, a buffer, a package insert describing information on an active ingredient, a container for storing the active ingredient, or a package.

Another embodiment of the present invention provides a therapeutic drug for a malignant tumor, comprising a HAT1 inhibitor, wherein the HAT1 inhibitor is used in combination with a KAT8 inhibitor. This therapeutic drug may be used to treat malignant tumors because HAT1 and KAT8 are inhibited.

Another embodiment of the present invention provides a therapeutic drug for a malignant tumor, comprising a KAT8 inhibitor, wherein the KAT8 inhibitor is used in combination with a HAT1 inhibitor. This therapeutic drug may be used to treat malignant tumors because HAT1 and KAT8 are inhibited.

As used herein, the wording "used in combination" means that a HAT1 inhibitor and a KAT8 inhibitor may be administered simultaneously or separately. In addition, the wording "used in combination" involves a dosage form where a HAT1 inhibitor and a KAT8 inhibitor are administered as a combination product. Further, the wording "used in combination" includes use during combination therapy. Meanwhile, regarding the order of administration, a HAT1 inhibitor may be first administered or a KAT8 inhibitor may be first administered. In addition, an embodiment of the present invention provides a combination product for treating a malignant tumor, comprising a HAT1 inhibitor and a KAT8 inhibitor. In addition, an embodiment of the present invention provides use of a HAT1 inhibitor in the manufacture of a malignant tumor therapeutic drug used when the HA1 inhibitor and a KAT8 inhibitor are used in combination.

An embodiment of the present invention provides a method for treating a malignant tumor, comprising the step of inhibiting HAT1 and KAT8 of a subject. In addition, an embodiment of the present invention provides a treatment method comprising the step of administering, to a subject, a HAT1 and KAT8 inhibitor, or a HAT1 inhibitor or a KAT8 inhibitor. In addition, an embodiment of the present invention provides use of a HAT1 and KAT8 inhibitor, or a HAT1 inhibitor or a KAT8 inhibitor in the manufacture of a therapeutic drug for a malignant tumor. The subject may be a patient who has already received the HAT1 inhibitor or the KAT8 inhibitor.

An embodiment of the present invention provides a growth inhibitor for a malignant tumor cell, comprising a HAT1 and KAT8 inhibitor, or a HAT1 inhibitor or a KAT8 inhibitor. An embodiment of the present invention provides a method for suppressing growth of a malignant tumor, comprising the step of inhibiting HAT1 and KAT8.

As used herein, the wording "a state in which cell growth is suppressed" includes a state in which the growth rate of test cells is significantly less than that before drug treatment. The growth rate can be evaluated by measuring the level of proliferation of cells during a given period of time. The level of proliferation may be measured, for example, visually or using absorbance as an index. Alternatively, the level of proliferation may be measured using, as an index, the level of a malignant tumor marker in a patient or a patient-derived sample.

As used herein, the term "treatment" includes exerting a prophylactic effect or a symptom-improving effect on a disease of a patient or on one or more symptoms involving the disease. As used herein, the "therapeutic drug" may be a pharmaceutical composition containing at least one pharmacologically acceptable carrier. The pharmaceutical composition can be produced by any process known in the art of drug formulation. Examples of the process include: mixing an active ingredient with the above carrier. In addition, the dosage form of the therapeutic drug is not limited as long as the therapeutic drug can be used for treatment. The pharmaceutical composition may be an active ingredient alone or a mixture of an active ingredient and any component. Further, examples of the dosage form of the above carrier include, but are not particularly limited to, a solid and a liquid (e.g., a buffer). Note that examples of the therapeutic drug for a malignant tumor include: a drug/agent (prophylactic) used for preventing a malignant tumor; an inducer for ameliorating a malignant tumor; and an inducer for producing a normal stem cell. The treatment includes treatment of an aging skin lesion and treatment of DNA damage caused by an anti-malignant tumor drug. This anti-malignant tumor drug may be, for example, a DNA damage-inducing anti-neoplastic agent.

A drug administration route effective in treatment is preferably used. Examples of the administration route include intravenous, subcutaneous, intramuscular, intraperitoneal, and oral administration. Examples of the dosage form may include an injection, a capsule, a tablet, and granules. When a polynucleotide is a therapeutic drug administered, use of an injection is effective. An aqueous solution for an injection may be stored in, for example, a vial or a stainless container. In addition, the aqueous solution for an injection may be combined with, for example, a saline solution, sugar (e.g., trehalose), NaCl, or NaOH. Further, the therapeutic drug may be formulated with, for example, a buffer (e.g., a phosphate buffer) and/or a stabilizer.

The single or daily dose may be, but is not limited to, 0.0001, 0.001, 0.01, 0.1, 1, 2, 3, 4, 5, 10, 50, 100, 150, 200 mg/kg body weight. The dose may be between any two of the above values. Alternatively, the single or daily dose may be 0.01, 0.1, 1, 2, 3, 4, 5, 6, 7, 8, 9, 10, 20, 30, 40, 50, 60, 70, 80, 90, 100, 200, 300, 400, 500, 600, 700, 800, 900, 1000, 5000, 10000, 15000, 20000, or 40000 mg/subject. The dose may be between any two of the above values. The dosage interval is not particularly limited, and may be, for example, 1, 2, 7, 10, 14, or 30 days between dosings. The dosage interval may be between any two of the above values. In addition, the dose, the dosage interval, and the administration method can be appropriately selected depending on the age, body weight, symptom, affected organ, etc., of a subject. In addition, an additional suitable chemotherapeutic agent may be used in combination during the administration. Further, the therapeutic drug preferably contains a therapeutically effective amount or a dose, which is effective in exerting a desired effect, of an active ingredient. One may judge that when the marker for a malignant tumor is decreased after dosing, there is a therapeutic effect.

From the viewpoints of safety and how to realize efficient treatment of malignant tumors, the dose is preferably set such that the blood concentration of an active ingredient after the active ingredient has been administered to a subject is less than or equal to the maximum effective blood concentration. Also, it is preferable that the dose is within an effective blood concentration. The effective blood concentration (from the minimum to maximum blood concentration inclusive) involves a blood concentration where a drug is effective and has a less adverse effect. As the effective blood concentration is used a value written on the interview form of a relevant medicine. Alternatively, the effective blood concentration may be calculated after the active ingredient is administered to humans to examine a dose where sufficient drug efficacy is exerted and any adverse effects are unlikely to occur. Further, the effective blood concentration may be calculated based on a value obtained through a clinical trial. The above therapeutic drug may have an identified maximum effective blood concentration for treatment applications (e.g., an antimicrobial agent) excluding therapeutic drugs for malignant tumors. The therapeutic drug may be administered such that the post-dosing blood concentration of an active ingredient is less than or equal to the above maximum effective blood concentration or within the effective blood concentration.

As used herein, the "maximum effective blood concentration" may be, for example, 0.01, 0.05, 0.1, 0.5, 1, 2, 3, 4, 5, 10, 20, 30, 40, 50, 60, 70, 80, 90, 100, 200, or 500 µg/ml. The concentration may be between any two of the above values. As used herein, the "minimum effective blood concentration" may be, for example, 0.001, 0.01, 0.1, 1, 10, 100, 500, 1000, 2500, 5000, 1.0 × 10⁴, 1.0 × 10⁵, 1.0 × 10⁶, or 1.0 × 10⁷ pg/ml. The concentration may be between any two of the above values.

The dose may be written in the interview form of each commercially available medicine (provided that the medicine has an indication other than an indication for malignant tumors) containing, as an active ingredient, chlorpropamide, vancomycin hydrochloride, betaxolol hydrochloride, colistin sulfate, bisoprolol fumarate, pinaverium bromide, oxprenolol hydrochloride, methylbenzethonium chloride, demecarium bromide, celiprolol hydrochloride, amikacin hydrate, or alprenolol hydrochloride (hereinafter, sometimes referred to as chlorpropamide, etc.) as described in below-described Example 2. The doses described in the interview forms include 500 mg/person, 2.0 g/person, 200 mg/person, 400,000 units, 10.2 mg/person, 200 mg/person, 300 mg/person, 120 mg/person, 10%, 0.25%, 198 mg/person, 400 mg/person, and 150 mg/person, respectively.

Note that the indications for the commercially available medicines containing chlorpropamide, etc., as an active ingredient include: type-2 diabetes mellitus; MRSA infection; essential hypertension, renal parenchymal hypertension, angina, glaucoma, ocular hypertension; *E. coli*, *Shigella*, multi-drug resistant *Pseudomonas aeruginosa*, infectious enteritis; essential hypertension, angina, ischemic heart disease, chronic heart failure based ion dilated cardiomyopathy; irritable bowel syndrome; tachyarrhythmia, angina; skin and/or mucosa infections; glaucoma; essential hypertension, renal parenchymal hypertension, angina; septic pneumonia, lung abscess, secondary infection of chronic respiratory disease, cystitis, pyelonephritis, peritonitis; angina, and tachyarrhythmia, respectively. In addition, the maximum effective blood concentrations (Cmax) described in the interview forms include 30 µg/ml, 30 µg/ml, 41.8 ng/ml, 4.4 µg/ml, 100 ng/ml, 1 µg/ml, 30 µg/µl, 0.3% (1.93 µg/mL), 0.25%, 300 ng/mL, 25 µg/mL, and 120 ng/mL, respectively.

The dose may be written in the interview form of each commercially available medicine (provided that the medicine has an indication other than an indication for malignant tumors) containing, as an active ingredient, fosinopril, clofilium tosylate, suprofen, esmolol hydrochloride, atractyloside potassium salt, imipenem, etifenin, cefixime, benzbromarone, cefoperazone dihydrate, pinacidil, nicergoline, oxethazaine, or antipyrine (hereinafter, sometimes referred to as fosinopril, etc.) as described in below-described Example 2. The doses described in the interview forms include 40.2 mg/person, 27.36 g/person, 10 g/day, 100 mg/person, 300 mg/person, 1.0 g/person, 400 mg/person, 100 mg/person, 150 mg/person, 4.0 g/person, 24 mg/person, 15 mg/person, 15 mg/person, and 1.0 g/person, respectively.

Note that the indications for the commercially available medicines containing fosinopril, etc., as an active ingredient include: hypertension, heart failure; heart failure, arrhythmias; inflammation, diseases with pain; arrhythmias, hypertension; diabetes mellitus, obesity; bacterial infections; arteriosclerosis, thrombosis; bacterial infections; hyperuricemia, gout; hypertension; cerebrovascular microangiopathy; peptic ulcer, reflux esophagitis, stomach pain; diseases with pain; headache, rheumatoid arthritis, and menstrual pain, respectively. In addition, the maximum effective blood concentrations (Cmax) described in the interview forms of the commercially available medicines containing fosinopril, etc., as an active ingredient include 4 µg/mL, 110 ng/mL, 1% external preparation, 2.7 µg/mL, 60 µg/mL, 82.8 µg/mL, 5.0 µg/mL, 3.97 µg/mL, 3.1 µg/mL, 1,000 ng/ml, 2.5 µg/mL, 96 ng eq./mL, 1 µg/mL, and 50 µg/mL, respectively.

The dose may be written in the interview form of each commercially available medicine (provided that the medicine has an indication other than an indication for malignant tumors) containing, as an active ingredient, merbromin, glycopyrrolate, metoprolol-(+,-)(+)-tartrate salt, pergolide mesylate, or bumetanide (hereinafter, sometimes referred to as merbromin, etc.) as described in below-described Example 2. The doses described in the interview forms include 100 mg/person, 50 µg/day, 120 mg/person, 252 µg/person, and 4.0 g/person, respectively.

Note that the indications for the commercially available medicines containing merbromin, etc., as an active ingredient include: infections; irritable bowel syndrome, preanesthetic treatment, bronchial obstruction; hypertension; Parkinson's disease; edema, and heart failure, respectively. In addition, the maximum effective blood concentrations (Cmax) described in the interview forms of the commercially available medicines containing merbromin, etc., as an active ingredient include 2% (external solution concentration), 40 ng/ml, 77 ng/ml, 26 µg/L, and 882 ng/ml, respectively.

The effect of treating malignant tumors may be evaluated by imaging, endoscopic examination, biopsy, or detection of a malignant tumor marker. One may judge that when the level of a marker in a subject or a subject-derived sample (e.g., a tissue, cells, a cell population, or blood) is significantly decreased after administration of a therapeutic drug, there is a therapeutic effect. At this time, the level of the marker after administration of the therapeutic drug may be 0.7, 0.5, 0.3, or 0.1 times the level before the administration (or of a control). Alternatively, one may judge that when the number of marker-positive cells in the subject-derived sample is significantly decreased after administration of the therapeutic drug, there is a therapeutic effect. At this time, the number of marker-positive cells after administration of the therapeutic drug may be 0.7, 0.5, 0.3, or 0.1 times the number before the administration (or of a control).

As used herein, the term "subject" includes a healthy individual, a study subject, or a patient. Examples of the subject include humans and non-human mammals (e.g., at least one of a mouse, guinea pig, hamster, rat, mouse, rabbit, pig, sheep, goat, cow, horse, cat, dog, marmoset, monkey, and chimpanzee). Meanwhile, the patient may be a patient who is determined or diagnosed as having the onset of a malignant tumor. In addition, the patient may be a patient who needs treatment of a malignant tumor. Examples of the patient include those having UV damage and those who need amelioration of UV-induced damage or DNA damage. In addition, the patient may be a subject who has received or will receive an anti-cancer drug. Note that the determination or diagnosis may be performed by imaging, endoscopic examination, biopsy, or detection of various markers.

An embodiment of the present invention provides a method for producing a stem cell, comprising the step of inhibiting HAT1 and KAT8. According to this production method, the stem cell can be easily produced. This production method does not necessarily require complicated manipulations, so that the efficiency and cost performance are excellent.

An embodiment of the present invention provides a sternness characteristic inducer comprising a HAT1 and KAT8 inhibitor. In addition, another embodiment of the present invention provides a sternness characteristic-inducing kit comprising a HAT1 inhibitor and a KAT8 inhibitor. In addition, another embodiment of the present invention provides a sternness characteristic inducer comprising a HAT1 inhibitor, wherein the HAT1 inhibitor is used in combination with a KAT8 inhibitor. In addition, another embodiment of the present invention provides a sternness characteristic inducer comprising a KAT8 inhibitor, wherein the KAT8 inhibitor is used in combination with a HAT1 inhibitor. In addition, another embodiment of the present invention provides a method for inducing a cell to express a sternness characteristic, comprising the step of inhibiting HAT1 and KAT8. According to them, the stem cell can be easily produced. In addition, another embodiment of the present invention provides a method for inducing a cell to express a sternness characteristic, comprising the step of causing the cell to contact a HAT1 and KAT8 inhibitor, or a HAT1 inhibitor or a KAT8 inhibitor. The induction of the sternness characteristic involves production of a stem cell.

Meanwhile, the above method may include a step of causing a cell or a cell population to contact a HAT1 and KAT8 inhibitor. The contact step may include an introduction step. Specifically, the above method may include a step of introducing a HAT1inhibitor and a KAT8 inhibitor into a cell or a cell population. In addition, the above method may include a step of collecting a stem cell or a stem cell population from the cell or the cell population containing the above inhibitors. In addition, the above method may include a step of detecting a stem cell marker. In addition, the above method may include a step of culturing the cell in a stem cell medium. In addition, the above method may include a step of culturing the cell in a medium containing a cell growth factor or a cell adhesion molecule. Examples of the cell growth factor include basic FGF, bFGF, and FGF-b. Examples of the cell adhesion molecule include VTN-N. The culture period may be, for example, 1, 2, 3, 4, 5, 6, 7, 8, 9, 10, 15, 20, 30, 40, 50, 60, 100 days or more. The period may be between any two of the above values. The above collection may be carried out with reference to, as an index, the cell morphology or gene expression. The above collection includes separation or isolation.

An embodiment of the present invention provides a process for producing a material for regenerative medicine, comprising the step of culturing the above stem cell. Examples of the material for regenerative medicine include organs for regenerative medicine. In addition, another embodiment provides a material for regenerative medicine, the material being obtainable by culturing a stem cell. In addition, another embodiment provides a method for treating a damaged tissue, comprising the step of injecting a stem cell or a stem cell population into a damaged site. In addition, another embodiment provides a therapeutic drug for treating a damaged tissue, comprising a stem cell or a stem cell population.

An embodiment of the present invention provides a method for producing a stem cell marker-expressing cell, comprising the step of inhibiting HAT1 and KAT8. Examples of the stem cell marker include Klf4, c-Myc, Oct4, Sox2, PROM1, Nanog, SSEA-1, ALP, eRas, Esg1, Ecat1, Fgf4, Gdf3, and REX-1. Examples of the stem cell marker include undifferentiation markers and pluripotent stem cell markers.

As used herein, the term "stem cell" includes a cell that can self-replicate and can differentiate into other types of cells. Examples of this stem cell include pluripotent stem cells. As used herein, the term "pluripotent stem cell" refers to a stem cell that is pluripotent. Examples of the pluripotent stem cell include cells expressing the equal or higher level of any of the undifferentiation markers than a human induced pluripotent stem cell, hiPSC (HPS0002 253G1). The induced pluripotent stem cells may be called iPS cells. Meanwhile, the stem cells created by the production method according to the above embodiment may express a high level of p53. Preferably, this level of expression of p53 is significantly higher than, for example, that of a control sample (e.g., an hiPSC (HPS0002 253G1), a p53-knockout cell, a normal cell, or a sample derived therefrom). In addition, this level of expression of p53 may be, for example, 1.05, 1.1, 1.2, 1.4, 1.6, 1.8, 2.0, 3.0, 4.0, 5.0, 10, 100, or 1000 times the level of expression of p53 in the control sample. A real-time PCR is a preferable method for measuring the level of expression of p53 in view of measurement accuracy and convenience.

As used herein, the "cell" may be a somatic cell. In addition, the above somatic cell may be derived from, for example, the skin, heart, liver, lung, stomach, intestine, kidney, uterus, aortic tunica adventitia, or mesenchymal tissues.

An embodiment of the present invention provides a method for increasing a percentage of a stem cell in a cell population, comprising the step of causing the cell population to contact a HAT1 and KAT8 inhibitor. In addition, another embodiment provides a process for producing a cell population having an increased percentage of a stem cell, comprising the step pf causing the cell population to contact a HAT1 and KAT8 inhibitor. In addition, another embodiment provides a method for increasing a percentage of a stem cell in a cell population, comprising the step of causing the cell population to contact a HAT1 and KAT8 inhibitor. In addition, another embodiment provides a process for producing a cell population having an increased percentage of a stem cell, comprising the step of causing the cell population to contact a HAT1 and KAT8 inhibitor.

As used herein, the term "cell population" refers to a population containing a plurality of cells. This cell population may be, for example, a population containing substantially uniform cells. In addition, the cell population may be a cell preparation. The cell preparation may contain, for example, cells and any buffer or medium component. The stem cell population may contain, for example, 80, 90, 95, 96, 97, 98, 99, or 100% of stem cells. The percentage may be between any two of the above values.

An embodiment of the present invention provides a research-use or medical kit comprising the above inhibitor(s). This kit may be used, for example, for preparing a stem cell, for preparing an artificial organ, or for treatment.

An embodiment of the present invention provides a screening method for selecting a stem cell inducer, a malignant tumor therapeutic drug, or a DNA damage-ameliorating agent, comprising the step of selecting a test substance that decreases expression or function of HAT1 or KAT8. This method may be used to efficiently obtain a stem cell inducer, a malignant tumor therapeutic drug, or a DNA damage-ameliorating agent. This method may comprise the steps of introducing a test substance into a cell and measuring a level of expression or function of HAT1 or KAT8.

As used herein, the wording "inhibiting the expression of a gene" means, for example, the inhibition of transcription of a gene to mRNA or the inhibition of translation of mRNA to a protein or polypeptide. In addition, the examples include the inhibition induced by decomposition of a gene, mRNA, or protein. In the field of biochemistry, examples of a role involving a gene include generating mRNA from the gene, producing a protein encoded by the gene, and causing the protein to exert its activity. Because of this, the wording "inhibiting the function of a gene" as used herein includes a decrease in the production level of mRNA or a protein after the expression of the gene is inhibited. Also, the wording "inhibiting the function of a gene" includes a decrease in the activity of the mRNA or protein encoded by the gene.

As used herein, the wording "a state in which the expression is inhibited" includes a state in which the level of expression is significantly decreased when compared with that in a normal state. The above wording "significantly decreased" may mean a state in which the level of expression is decreased to 0.4, 0.35, 0.3, 0.25, 0.2, 0.15, 0.1, 0.05, 0.01, or 0 times the original level. The wording may mean a state in which the level of expression is decreased to X times the original level (X is between any two of the above values). From the viewpoints of efficiently suppressing growth of malignant tumor cells or stably producing a stem cell, 0.2 or less times the original level is preferable and 0.1 or less times the original level is more preferable. Note that the level of expression may be determined by using, as an index, the level of mRNA or protein. In addition, as used herein, the term "significantly" may include a case of p < 0.05 when Student's t test (one-sided or two-sided) is used to evaluate a statistically significant difference. Also, the term may include a state in which there is a substantial difference. Note that the intensity of inhibition with respect to a "state in which the function is inhibited" may also refer to the intensity of inhibition with respect to the inhibition of expression in substantially the same manner as in some embodiments.

As used herein, examples of the "inhibitor" form include, but are not particularly limited to, polynucleotides (e.g., an RNA strand(s), a DNA strand(s)), low-molecular-weight compounds (e.g., a low-molecular-weight organic compound), antibodies, polypeptides (e.g., an enzyme), and compositions containing the above molecule(s). The RNA strand(s) may be a translation inhibitory RNA against HAT1 or KAT8. Examples of this RNA species include RNA species with a function of inhibiting translation from gene to protein. Examples of this RNA species include RNAi molecules and miRNA-related molecules. The function of inhibiting translation may be checked by quantifying the level of expression of a corresponding RNA strand by real-time RT-PCR. Alternatively, to check the function, the level of expression of the RNA strand may be analyzed by using Northern blotting and/or the level of the resulting protein may be analyzed by using Western blotting as well as the resulting phenotype may be observed. In addition, a plasmid for generating an RNA molecule that can inhibit translation of a specific gene may be purchased from a service company (e.g., TAKARA BIO INC.). As the above DNA strand can be used a DNA strand encoding the above RNA strand. The form of the DNA strand may be, for example, a vector. Any of the above antibodies may be produced using a known antibody production protocol (e.g., the method described in Clackson et al., Nature. 1991 Aug 15; 352(6336): 624-628) or may be purchased from a service company (e.g., Abcam plc). At this time, an antibody library may be constructed, and it is preferable to select an antibody with increased inhibition activity. Any of the polypeptides may be purchased from a service company (e.g., Wako Pure Chemical Industries, Ltd.).

The above low-molecular-weight compounds may be obtained, for example, by FDA library screening or by combinatorial chemistry and/or HTS (high-throughput screening). For the combinatorial chemistry, an automated synthesizer, an L-COS series (SHOKO, Inc.), for example, may be used. For the HTS, Octet system (ForteBio, Inc.), for example, may be used. The inhibitor screening may include, for example, a step of causing a test substance to contact a test cell to examine a level of expression of mRNA or function of a protein and a step of determining that when the level of expression or the function is decreased to 0.2 or less times the original level, the test substance is an inhibitor. From the viewpoints of effectively suppressing growth of malignant tumor cells, efficiently producing a stem cell, or effectively ameliorating DNA damage, preferable examples of the above low-molecular-weight compounds include: at least one compound selected from the group consisting of chlorpropamide, vancomycin, betaxolol, colistin, bisoprolol, pinaverium bromide, oxprenolol, methylbenzethonium chloride, demecarium bromide, celiprolol, amikacin, and alprenolol; salts thereof; and solvates thereof. From the viewpoint of inhibiting HAT1, preferable examples of the above low-molecular-weight compounds include: at least one compound selected from the group consisting of fosinopril, clofilium, suprofen, esmolol, atractyloside, imipenem, etifenin, cefixime, benzbromarone, cefoperazone, pinacidil, nicergoline, oxethazaine, and antipyrine; salts thereof; and solvates thereof. From the viewpoint of inhibiting KAT8, preferable examples of the above low-molecular-weight compounds include: at least one compound selected from the group consisting of merbromin, glycopyrrolate, metoprolol, pergolide, and bumetanide; salts thereof; and solvates thereof.

Meanwhile, regarding the intermolecular binding strength between a subject inhibitor and HAT1 or KAT8, the Avg K_{D} (M) may be 1.0 × 10⁻⁵, 5.0 × 10⁻⁶, 1.0 × 10⁻⁶, 5.0 × 10⁻⁷, 1.0 × 10⁻⁷, 5.0 × 10⁻⁸, 1.0 × 10⁻⁸, 5.0 × 10⁻⁹, 1.0 × 10⁻⁹, 1.0 × 10⁻¹⁰, 1.0 × 10⁻¹¹, 1.0 × 10⁻¹², or less. The Avg K_{D} may be between any two of the above values. From the viewpoints of efficiently suppressing growth of malignant tumor cells, stably producing a stem cell, or increasing a rate of ameliorating DNA damage, this value is preferably 5.0 × 10⁻⁶ or less, more preferably 1.0 × 10⁻⁷ or less, still more preferably 1.0 × 10⁻⁸ or less, and still more preferably 5.0 × 10⁻⁹ or less. In addition, regarding the intermolecular binding strength between a subject inhibitor and HAT1 or KAT8, the Avg Kₒₙ (1/Ms) may be 5.0 × 10, 1.0 × 10², 5.0 × 10², 1.0 × 10³, 5.0 × 10³, 1.0 × 10⁴, 5.0 × 10⁴, 1.0 × 10⁵, 5.0 × 10⁵, 1.0 × 10⁶, or higher. The Avg Kₒₙ may be between any two of the above values. From the viewpoints of efficiently suppressing growth of malignant tumor cells, stably producing a stem cell, or increasing a rate of ameliorating DNA damage, this value is preferably 1.0 × 10² or higher, more preferably 1.0 × 10³ or higher, still more preferably 5.0 × 10³ or higher, and still more preferably 1.0 × 10⁴ or higher. In addition, regarding the intermolecular binding strength between a subject inhibitor and HAT1 or KAT8, the Avg K_{off} (1/s) may be 1.0 × 10⁻², 5.0 × 10⁻³, 1.0 × 10⁻³, 5.0 × 10⁻⁴, 1.0 × 10⁻⁴, 5.0 × 10⁻⁵, 1.0 × 10⁻⁵, 5.0 × 10⁻⁶, 1.0 × 10⁻⁶, 1.0 × 10⁻⁷, 1.0×10⁻⁸, or less. The Avg K_{off} may be between any two of the above values. From the viewpoints of efficiently suppressing growth of malignant tumor cells, stably producing a stem cell, or increasing a rate of ameliorating DNA damage, this value is preferably 5.0 × 10⁻³ or less, more preferably 1.0 × 10⁻³ or less, still more preferably 4.0 × 10⁻⁵ or less, and still more preferably 5.0 × 10⁻⁵ or less. The intermolecular binding strength may be measured by, for example, surface plasmon resonance analysis. For the surface plasmon resonance analysis, a Biacore system (e.g., Biacore T100), for example, may be used. Alternatively, one may ask a service company to analyze the intermolecular binding strength.

From the viewpoints of effectively suppressing growth of malignant tumor cells, efficiently producing a stem cell, or effectively ameliorating DNA damage, the subject inhibitor is preferably a translation inhibitory RNA species or a low-molecular-weight compound. The inhibitor contains a substance that inhibits the expression or function of a target. Preferably, the inhibitor is less toxic or a substance with substantially no cell toxicity. In this case, when the inhibitor is administrated *in vivo*, the adverse effects can be suppressed. Note that when particularly indicated, hsa-miR-520d-5p, nucleic acid containing the mature miRNA thereof, nucleic acid substantially equivalent thereto, or nucleic acid from which the former is expressed may be excluded from the form of the inhibitor.

As used herein, examples of the "drug/agent" include therapeutic drugs, quasi drugs, external medicines, beauty care preparation, cosmetics, or medical cosmetics. This drug/agent may be a composition comprising an active ingredient and one or more pharmaceutically or cosmetically acceptable carriers. The dose, the dosing interval, and the dosing method can be appropriately selected depending on the age, body weight, symptom, affected site, etc., of a subject. The drug/agent may be used *in vitro* or *in vivo.* The drug/agent preferably contains a therapeutically effective amount, a cosmetically effective amount, or a dose, which is effective in exerting a desired effect, of an active ingredient.

The above inhibitor can be introduced into cells and the cells can be cultured in accordance with a method known in the art. Examples of the introduction method include viral vector-mediated infection, a calcium phosphate method, lipofection, electroporation, and microinjection. In addition, drug resistance and a cell sorter, for example, may be used to select only the cell into which the inhibitor has been introduced. The relevant medium may be a medium containing, for example, a cell growth factor. In addition, examples of the medium used may include media for keeping cells undifferentiated (e.g., pluripotent stem cell medium, ReproStem medium, primate ES cell medium (COSMO BIO Co., Ltd.)) and regular human cell media (e.g., DMEM or RPMI-based media). For instance, the 10% FBS-containing ReproStem medium may be used in culture conditions at 37°C and 5% CO₂. These numbers may be subject to change within +/- 10, 20, or 30%. The cells may be established or cultured in the absence of feeder cells. When the above RNA strand(s) is introduced into a cell, a plurality of RNA strands may be combined and introduced into the cell. Further, when the above DNA strand(s) is introduced into a cell, a plurality of DNA strands may be introduced into the cell. Here, the number of the "plurality of RNA strands" or "plurality of DNA strands" may be 2, 3, 4, 5, 10, or 20.

As used herein, examples of the "vector" include: viral vectors (e.g., lentivirus, adenovirus, retrovirus, or HIV vectors); *E*. *coli*-derived plasmids (e.g., pBR322); *Bacillus subtilis*-derived plasmids (e.g., pUB110); yeast-derived plasmids (e.g., pSH19); bacteriophages such as λ phage; and psiCHECK-2, pA1-11, pXT1, pRc/CMV, pRc/RSV, pcDNAI/Neo, pSUPER (OligoEngine, Inc.), BLOCK-it Inducible H1 RNAi Entry Vector(Invitrogen, Inc.), and pRNATin-H1.4/Lenti (GenScript, Corp., NJ, USA). The above vectors may each contain, for example, a promoter, a replication origin, and/or an antibiotic resistance gene, which are essential components for expression of the DNA strand. Each vector may be what is called an expression vector.

Meanwhile, from the viewpoint of stably inhibiting expression of HAT1 or KAT8, the above RNA strand(s) is preferably a translation inhibitory RNA species for which a portion of HAT1 mRNA or MAT8 mRNA is a target sequence. In addition, from the viewpoint of stably inhibiting expression of HAT1 and KAT8, each RNA strand preferably contains a nucleotide sequence complementary to a portion of HAT1 mRNA or KAT8 mRNA. Further, the above RNA strand(s) may contain a guide strand. The above "portion" may mean 5, 10, 15, 16, 17, 18, 19, 20, 21, 22, 23, 24, 25, 26, 27, 28, 29, 30, 35, 40, 50 nucleotides or more. The number may be between any two of the above values. In below-described Example 1, used was a shRNA lentivirus vector set (the inhibition of each of HAT1 and KAT8 has been guaranteed).

HAT1 shRNA-expressing lentivirus particles (psi-LVRU6GP for HAT1 mixture; Genecopoeia, Inc.), which were used in below-described Example 1, can be generated from 4 different plasmids, each encoding shRNA against the target gene. Their catalog numbers are HSH021144-31-LVRU6GP, HSH021144-32-LVRU6GP, HSH021144-33-LVRU6GP, and HSH021144-34-LVRU6GP. These four different plasmids can generate four different shRNAs. The target sequences for the four different shRNAs are SEQ ID NOS: 10 (5'-CCAACACAGCAAUUGAACUAA-3'), 11 (5'-GCCAUUCGGAACCUUACUUCA-3'), 12 (5'-GCUUUCGAGAAUAUCAUGAAA-3'), and 13 (5'-GCUAGAAGGGUUUAUGAAAUU-3'), respectively. When siRNAs are generated from the four different shRNAs, each guide strand contains the nucleotide sequence set forth in SEQ ID NO: 14 (GGUUGUGUCGUUAACUUGAUU), 15 (CGGUAAGCCUUGGAAUGAAGU), 16 (CGAAAGCUCUUAUAGUACUUU), or 17 (CGAUCUUCCCAAAUACUUUAA), respectively. In addition, each passenger strand contains the nucleotide sequence set forth in SEQ ID NO: 18, 19, 20, or 21, respectively.

KAT8 shRNA-expressing lentivirus particles (psi-LVRU6GP for KAT8 mixture; Genecopoeia, Inc.), which were used in below-described Example 1, can be generated from 4 different plasmids, each encoding shRNA against the target gene. Their catalog numbers are HSH020510-31-LVRU6GP, HSH020510-32-LVRU6GP, HSH020510-33-LVRU6GP, and HSH020510-34-LVRU6GP. These four different plasmids can generate four different shRNAs. The target sequences for the four different shRNAs are SEQ ID NOS: 22 (5'-CCGAGAGGAAUUCUAUGUACA-3'), 23 (5'-GGGAACUACGAAAUUGAUGCC-3'), 24 (5'-CACCAAGGUGAAGUAUGUGGA-3'), and 25 (5'-GUACUGCCUCAAGUACAUGAA-3'), respectively. When siRNAs are generated from the four different shRNAs, each guide strand contains the nucleotide sequence set forth in SEQ ID NO: 26 (GGCUCUCCUUAAGAUACAUGU), 27 (CCCUUGAUGCUUUAACUACGG), 28 (GUGGUUCCACUUCAUACACCU), or 29 (CAUGACGGAGUUCAUGUACUU), respectively. In addition, each passenger strand contains the nucleotide sequence set forth in SEQ ID NO: 30, 31, 32, or 33, respectively.

An embodiment of the present invention provides the nucleotide sequences set forth in SEQ ID NOS: 10 to 33 such that as long as a desired effect is exerted, (a) a nucleotide sequence containing one or more nucleotide deletions, substitutions, insertions, or additions in any of the above nucleotide sequences may be permitted; and (b) at least one nucleotide sequence selected from the group consisting of nucleotide sequences having 90% or more homology to any of the above nucleotide sequences may be permitted.

As used herein, the above "number of nucleotides" may be, for example, 10, 8, 6, 5, 4, 3, or 2. The number may be any of the values or less. From the viewpoints of effectively suppressing growth of malignant tumor cells, efficiently producing a stem cell, or effectively ameliorating DNA damage, the number is preferably 3 or less and more preferably 2 or less.

As used herein, the term "90% or more" may mean that the number is, for example, 90, 95, 96, 97, 98, 99, or 100%. The number may be between any two of the above values. The above term "homology" may refer to a ratio of the number of identical nucleotides between two or among a plurality of nucleotide sequences to the total number of nucleotides as calculated in accordance with a method known in the art. Before the calculation of the ratio, nucleotide sequences selected from the group of nucleotide sequences compared are aligned. If the ratio regarding the identical nucleotides needs to be optimized, gaps are inserted in some portions of the nucleotide sequences. An alignment method, a ratio calculation method, a comparison method, and a related computer program have been conventionally well-known in the art (e.g., BLAST, GENETYX). The homology (%) may be represented using values obtained by Blastn with default settings. Alternatively, the homology (%) may be calculated using the formula (the number of identical nucleotides / the total number of nucleotides in a sequence compared) × 100).

As used herein, the term "polynucleotide" may include nucleotides or bases or those having a plurality of their equivalents. The concept of the polynucleotide includes, for instance, a chemically-modified polynucleotide, a salt of a polynucleotide, a solvate of a polynucleotide, and a polynucleotide that can bind to a chemical. Examples of the chemical modification include methylation. Examples of the chemical include cellular uptake enhancers (e.g., PEGs or derivatives thereof), labeled tags (e.g., fluorescently labeled tags), and linkers (e.g., nucleotide linkers). As used herein, the concept of the nucleotide includes, for instance, RNA or DNA nucleotides with/without chemical modification. Examples of the equivalents include nucleotide analogs. Examples of the nucleotide analogs include synthetic nucleotides. Examples of the "RNA strand" include a structure in which two or more RNA nucleotides with/without chemical modification or equivalents thereof are bonded. Examples of the polynucleotide include single-strand or double-strand polynucleotides. A nucleotide sequence may be represented by using, for instance, A (adenine), G (guanine), C (cytosine), and T (thymine). Meanwhile, T and U (uracil) are switchable depending on their usage. The nucleotides in such a nucleotide sequence may include A, G, C, and T with/without chemical modification.

The number of nucleotides in the polynucleotide may be, for instance, 15, 16, 17, 18, 19, 20, 21, 22, 23, 24, 25, 30, 40, 50, 60, 70, 80, 90, 100, 200, or 500. The number may be between any two of the above values. The polynucleotide can be synthesized using, for example, a DNA/RNA synthesizer. In addition, the polynucleotide can be purchased from any of service companies (e.g., Invitrogen, Inc., TAKARA BIO INC.) where DNA or RNA polynucleotides can be synthesized. Further, the vector can be synthesized by using service from each company.

As used herein, examples of the "miRNA-related molecules" include miRNA, pri-miRNA, and pre-miRNA. The miRNA-related molecules are known to contribute to inhibiting translation of a target RNA strand. In addition, the translation inhibition is known to be caused by miRNA having an imperfect complementary strand that contains a mismatch(es) and binds partially to the target.

As used herein, the "RNAi molecules" are RNA strands that function as RNAi, and examples include small RNAs that function as siRNA, shRNA, or RNAi. The RNAi molecules can be designed by using, for instance, siDirect 2.0 (Naito et al., BMC Bioinformatics., 2009, Nov. 30; 10:392). In addition, the RNAi molecules may be manufactured by using service from any of service companies (e.g., TAKARA BIO INC.).

As used herein, the "RNAi" involves phenomena such that at least one of siRNA, shRNA, short or long, single or double strand RNA, or a derivative thereof is used to inhibit or silence the function of a target gene or mRNA, etc.

As used herein, the "siRNA" includes an RNA strand(s) that can induce RNAi. Generally speaking, the siRNA double strand may be separated into a guide strand and a passenger strand. Then, the guide strand is incorporated into a RISC. The guide strand incorporated into the RISC is used for recognition of a target RNA. Meanwhile, synthetic RNA is primary used in RNAi research. However, it has been known that endogenous counterparts exist *in vivo.* Each RNA strand may be composed of RNA having 15 or more nucleotides. If the number of nucleotides is 15 or more, the RNA is likely to bind specifically to a target polynucleotide. In addition, each RNA strand may be composed of RNA having 40 or less nucleotides. When the number of nucleotides is 40 or less, there is a lower risk of disadvantageous phenomena such as interferon responses etc.

As used herein, the term "shRNA" includes a single RNA strand that can induce RNAi and can produce a structure (a hairpin-like structure) in which the RNA is bent like a hairpin. Usually, the shRNA is cleaved by a Dicer in a cell to yield siRNA. This siRNA is known to cause a target RNA to be cut. The above shRNA may be composed of 35 or more nucleotides. If the number of nucleotides is 35 or more, a particular hairpin-like structure is likely to be specifically formed in the shRNA. In addition, the above shRNA may be composed of RNA having 100 or less nucleotides. When the number of nucleotides is 100 or less, there is a lower risk of disadvantageous phenomena such as interferon responses etc. In this connection, many pre-miRNAs, the structure and function of which are similar to those of common shRNA, have about 100 or more nucleotides. Accordingly, the length of shRNA may not be 100 bp or less. Nevertheless, the shRNA should be functional.

As used herein, the "small RNA" refers to a relatively small RNA strand(s), and examples include siRNA, shRNA, miRNA-related molecules, antisense RNA, and single or double strand, low-molecular-weight RNA.

Translation inhibitory RNA species may contain a 1 to 5-nucleotide overhang at the 5' or 3' end from the viewpoint of increasing the efficiency of inhibiting translation. The number of nucleotides may be 5, 4, 3, 2, or 1. The number may be between any two of the above values. When the translation inhibitory RNA species is double-stranded, a mismatch RNA may be present between the two RNA strands. The number of mismatches may be 1, 2, 3, 4, 5, or 10 or less. The number may be between any two of the above values. The translation inhibitory RNA species may contain a hairpin loop. The number of nucleotides in the hairpin loop may be, for example, 10, 8, 6, 5, 4, or 3. The number may be between any two of the above values. Note that regarding the assignment of each nucleotide sequence, the left side is the 5' end and the right side is the 3' end.

The length of the translation inhibitory RNA species may be, for example, 15, 16, 17, 18, 19, 20, 21, 22, 23, 24, 25, 30, 40, 50, 60, 70, 80, 90, 100, 200, or 500 nucleotides. The number may be between any two of the above values. The length is preferably 15 or more and 100 or less nucleotides from the viewpoint of increasing the translation inhibition efficiency.

An embodiment of the present invention provides a DNA damage-ameliorating agent comprising a HAT1 and KAT8 inhibitor. This DNA damage-ameliorating agent can ameliorate DNA damage. As described in Examples, after cells that were subject to lethal DNA damage caused by UV irradiation were treated with a low-molecular-weight compound that inhibits HAT1 and KAT8, the DNA damage was markedly ameliorated.

It is preferable that this DNA damage-ameliorating agent is used after a subject is irradiated with UV light from the viewpoint of increasing the DNA damage-ameliorating effect. It is also preferable that this DNA damage-ameliorating agent is used in night from the viewpoint of increasing the DNA damage-ameliorating effect. The night includes a period from sunset to bedtime or a period from 18 to 26 o'clock. The period from 18 to 26 o'clock may mean 18, 19, 20, 21, 22, 23, 24, 25, or 26 o'clock. The time may be between any two of the above values.

As used herein, the "ameliorating agent" may be a composition for beauty care use. In addition, examples of the composition for beauty care use include compositions used for cosmetics. In addition, the ameliorating agent may be a composition for research or regenerative medicine use. In addition, the ameliorating agent may be a composition for treatment. In addition, examples of the damage-ameliorating agent include compositions for repairing damage. Examples of the composition for repair include compositions for promoting the repair.

In addition, the DNA damage-ameliorating agent may be a composition for transdermal absorption. The DNA damage-ameliorating agent may be applied onto the skin. Examples of the dosage form of the DNA damage-ameliorating agent include creams and liquids.

As used herein, the "DNA damage" includes a cleavage of single-strand DNA, a cleavage of double-strand DNA, and a DNA nucleotide mutation. How much the composition ameliorates DNA damage may be evaluated by determining how much the cell growth, which has been decreased by the DNA damage, is increased by treatment using the composition. Alternatively, commercially available kits (e.g., OxiSelect (COSMO BIO co., ltd.), CometAssay Kit (Funakoshi Co., Ltd.)) may be used for the evaluation.

As used herein, the "UV light" includes an electromagnetic wave with a wavelength of from 10 to 400 nm. The wavelength may be, for example, 10, 50, 100, 200, 250, 280, 300, 315, 350, or 400. The wavelength may be between any two of the above values. From the viewpoint of promoting beauty, it is preferable to ameliorate damage caused by near ultraviolet radiation (200 to 380 nm). The near ultraviolet radiation may be divided into UVA (315 to 400 nm), UVB (280 to 315 nm), and UVC (less than 280 nm). The UV irradiation dose may be, for example, 0.01, 0.1, 0.5, 1, 5, 15, 20, 25, or 30 J/cm². The dose may be between any two of the above values.

As used herein, the term "beauty care use" includes making one's looks beautiful. Examples of the beauty care use include that the skin damaged by UV irradiation is conditioned.

In an embodiment of the present invention, the "research-use" or "regenerative medicine-use" composition may be used to ameliorate damage or growth inhibition of materials (e.g., cells or tissues) used, for example, in "research" or "regenerative medicine", which damage or growth inhibition has been caused by UV irradiation or a DNA damage-inducing chemical. This enables research or regenerative medicine materials to be efficiently used.

An embodiment of the present invention provides a UV damage-ameliorating agent comprising a HAT1 and KAT8 inhibitor, or a HAT1 inhibitor or a KAT8 inhibitor. This UV damage-ameliorating agent can ameliorate UV-induced cell damage. This UV damage-ameliorating agent can exert an effect such that cells which are subject to substantial cell death caused by UV irradiation are recovered from UV-induced damage.

An embodiment of the present invention provides a beauty care preparation or a cosmetic comprising a HAT1 and KAT8 inhibitor, or a HAT1 inhibitor or a KAT8 inhibitor. The beauty care preparation or cosmetic can ameliorate UV-induced cell damage. The beauty care preparation or cosmetic may comprise, for example, a whitening component (e.g., arbutin), a moisturizing component (e.g., vaseline, sodium hyaluronate), or a skin-beautifying component (e.g., vitamin C). The beauty care preparation or cosmetic may be included in, for instance, a beauty care or cosmetic container. Examples of the beauty care or cosmetic container include tubes, pump-type containers, wide mouth jars, and narrow mouth bottles.

An embodiment of the present invention provides a beauty care method comprising the step of administering, to a subject, a composition comprising a HAT1 and KAT8 inhibitor, or a HAT1 inhibitor or a KAT8 inhibitor. This method can be used to ameliorate the subject's cells with DNA damage. As used herein, the term "amelioration" involves an effect of making a damaged state as close to an original state as possible, and examples include repair and treatment. The step of administering a dug/agent to a subject may include a step of administering the drug/agent to the subject's skin. In addition, the beauty care method may further comprise a step of washing the skin or a step of disinfecting the skin.

An embodiment of the present invention provides a method for ameliorating DNA damage, comprising the step of administering, to a subject, a HAT1 and KAT8 inhibitor, or a HAT1 inhibitor or a KAT8 inhibitor. In addition, an embodiment of the present invention provides a method for ameliorating UV damage, comprising the step of administering, to a subject, a HAT1 and KAT8 inhibitor, or a HAT1 inhibitor or a KAT8 inhibitor. The administration step may include a step of applying the inhibitor(s) onto the subject's skin. In addition, any of the above methods may further include a step of administering the composition to the skin, a step of washing the skin, or a step of disinfecting the skin. In addition, any of the above methods may further include a step of administering an anti-cancer drug to the subject. An embodiment of the present invention provides use of a HAT1 and KAT8 inhibitor, or a HAT1 inhibitor or a KAT8 inhibitor in the manufacture of a DNA damage-ameliorating agent or a UV damage-ameliorating agent.

An embodiment of the present invention provides a method for ameliorating DNA damage or UV damage, comprising the step of causing a HAT1 and KAT8 inhibitor, or a HAT1 inhibitor or a KAT8 inhibitor to contact a material for research or regenerative medicine use.

An embodiment of the present invention provides a malignant tumor therapeutic drug, sternness characteristic inducer, or DNA damage-ameliorating agent comprising: at least one compound selected from the group consisting of chlorpropamide, vancomycin, betaxolol, colistin, bisoprolol, pinaverium bromide, oxprenolol, methylbenzethonium chloride, demecarium bromide, celiprolol, amikacin, and alprenolol; a salt thereof; or a solvate thereof. They can treat malignant tumors, induce sternness characteristics, or ameliorate DNA damage.

An embodiment of the present invention provides a HAT1 and KAT8 inhibitor comprising: at least one compound selected from the group consisting of chlorpropamide, vancomycin, betaxolol, colistin, bisoprolol, pinaverium bromide, oxprenolol, methylbenzethonium chloride, demecarium bromide, celiprolol, amikacin, and alprenolol; a salt thereof; or a solvate thereof. This inhibitor can inhibit HAT1 and KAT8.

From the viewpoint of increasing a rate of inhibiting HAT1 and KAT8, this inhibitor preferably comprises at least one compound selected from chlorpropamide, vancomycin hydrochloride, betaxolol hydrochloride, colistin sulfate, bisoprolol fumarate, pinaverium bromide, oxprenolol hydrochloride, methylbenzethonium chloride, demecarium bromide, celiprolol hydrochloride, amikacin hydrate, and alprenolol hydrochloride.

As used herein, examples of the "salt" include, but are not particularly limited to, anionic salts that are formed using any acidic group (e.g., carboxyl) and cationic salts that are formed using any basic group (e.g., amino). Examples of the salts include inorganic salts, organic salts, and salts disclosed in the article (Berge, Bighley, and Monkhouse, J. Pharm. Sci., 1977, 66, 1-19). The examples further include metal salts, ammonium salts, salts of an organic base, salts of an inorganic acid, salts of an organic acid, and salts of a basic or acidic amino acid. Examples of the metal salts include alkali metal salts (e.g., sodium salts, potassium salts), alkali earth metal salts (e.g., calcium salts, magnesium salts, barium salts), and aluminum salts. Examples of the salts of an organic base include salts of trimethylamine, triethylamine, pyridine, picoline, 2,6-lutidine, ethanolamine, diethanolamine, triethanolamine, cyclohexylamine, dicyclohexylamine, or N,N'-dibenzylethylenediamine. Examples of the salts of an inorganic acid include salts of hydrochloric acid, hydrobromic acid, nitric acid, sulfuric acid, or phosphoric acid. Examples of the salts of an organic acid include salts of formic acid, acetic acid, trifluoroacetic acid, phthalic acid, fumaric acid, mesylic acid, tosylic acid, oxalic acid, tartaric acid, maleic acid, citric acid, succinic acid, malic acid, methanesulfonic acid, benzenesulfonic acid, or p-toluenesulfonic acid. Examples of the salts of a basic amino acid include salts of arginine, lysine, or ornithine. Examples of the salts of an acidic amino acid include salts of aspartic acid or glutamic acid.

As use herein, the term "solvate" refers to a compound formed using a solute and a solvent. J. Honig et al., The Van Nostrand Chemist's Dictionary P650 (1953) can be consulted regarding the solvate. If the solvent is water, the solvate formed is a hydrate. Preferably, the solvent does not interfere with the biological activity of the solute. Examples of such a preferable solvent include, but are not limited to, water, ethanol, and acetic acid. The most preferred solvent is water (including a monohydrate, a dihydrate, and a trihydrate). A compound or a salt thereof according to an embodiment of the present invention absorbs moisture when contacting the air or recrystallized. They may have hygroscopic moisture or become a hydrate.

Any documents and (patent or patent application) publications, which are cited herein, are incorporated by reference in its entirety.

As used herein, the term "or" may be used when "at least one" matter listed in the text of specification can be employed. The same applies to the term "or". As used herein, when the wording "between any two of the above values" is indicated, the two values are inclusive in the range. As used herein, the phrase "from A to B" means "A or more and B or less".

As described above, the embodiments of the present invention have been illustrated. These embodiments are examples of the present invention. Accordingly, various configurations other than the above embodiments can be adopted. In addition, combinations among the above-described embodiments can also be employed.

### Examples

Hereinafter, the present invention is further illustrated by referring to Examples. The present invention, however, is not limited to them.

### <Example 1>

### 1.1. Inhibition by shRNAs against HAT1 and KAT8

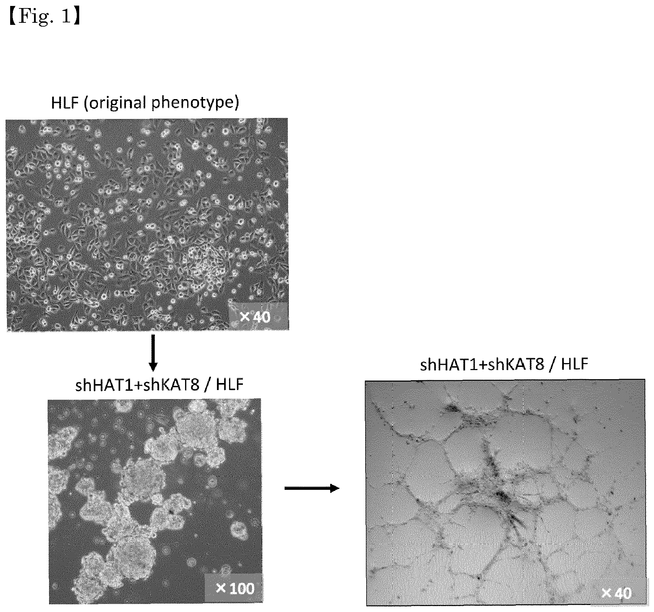

HLF cells (liver cancer cells) were infected with both HAT1 shRNA-expressing lentivirus particles (psi-LVRU6GP for HAT1 mixture, Genecopoeia, Inc.) and KAT8 shRNA-expressing lentivirus particles (psi-LVRU6GP for KAT8 mixture, Genecopoeia, Inc.) (hereinafter, sometimes referred to as shHAT1/shKAT8) at MOI = 1. Then, the levels of expression of target genes were examined by RT-PCR. The results demonstrated a significant inhibitory effect on the HAT1 and KAT8 genes. FIG. 1 shows the cell morphology of the shHAT1/shKAT8-containing HLF cells after cultured for 7 days in RPMI1640 medium. The shRNA-containing HLF cells are transformed into cells with a spherical shape (the lower left panel). After additional 7 days of culturing, the morphology of the HLF cells looked like a unique radial network, which was completely different from that of original HLF cells. This demonstrated that the sternness characteristics of HLF cells are induced and the HLF cells were then differentiated.

In addition to the above experiment, shRNA against either the HAT1 gene or the KAT8 gene alone was infected into HLF cells. The results, however, showed no significant change in cell morphology after only one of them was given.

### 1.2. Cell Morphology

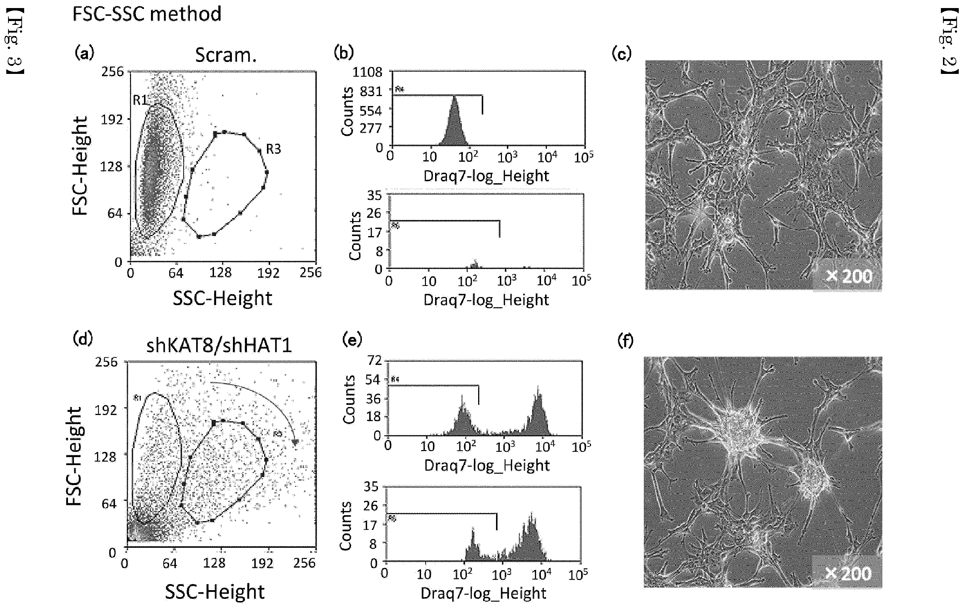

The shapes of the shHAT1/shKAT8-containing HLF cells after cultured for 7 days in RPMI1640 medium were analyzed using a cell sorter. The lower panels of FIG. 2 show the results. The upper panels of FIG. 2 show the case where a scrambled control (control nucleic acid) was introduced into HLF cells. This analysis shows the results of measuring the forward and side scatter of the cells. If the cells are spherical, the cell population likely appears in the red arc (arrowed) direction in the lower panel (d). In addition, the right-side peaks in the panel (e) mean that there were spherical fat droplets. This experiment demonstrated that most of the shHAT1/shKAT8-containing HLF cells were spherical.

### 1.3. Analysis of Expression of Stem Cell Markers

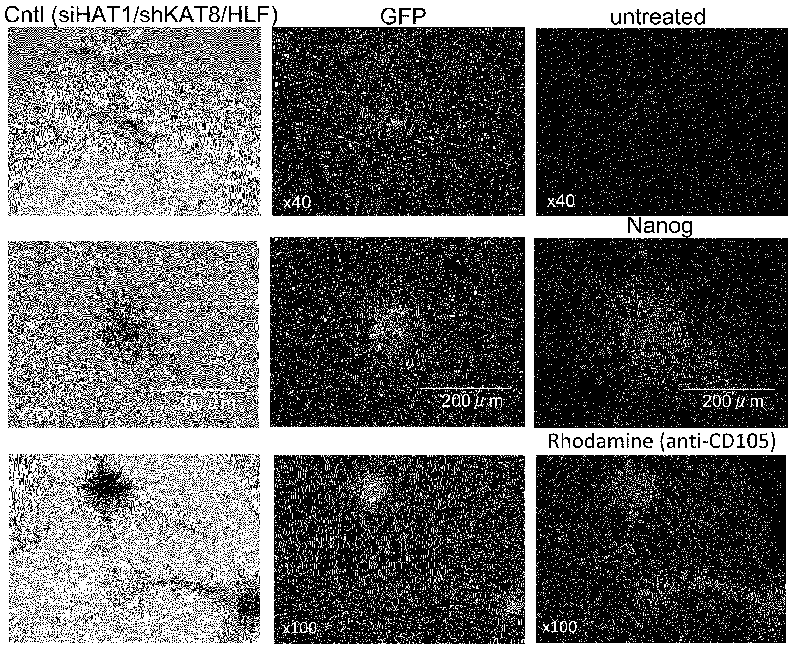

The shHAT1/shKAT8-containing HLF cells were cultured in RPMI1640 medium for 7 days and were then cultured in mesenchymal stem cell medium for additional 7 days to examine the levels of expression of relevant proteins in the cells by immunostaining. FIG. 3 shows the results. A pluripotent cell marker Nanog and a mesenchymal stem cell marker CD105 were expressed. This indicates that the shHAT1/shKAT8-containing HLF cells were turned into cells different from the original HLF cells and were transformed into stem cell-like cells. The GFP expression is an indicator for the vector-containing cells.

### 1.4. Cell Transformation and Differentiation

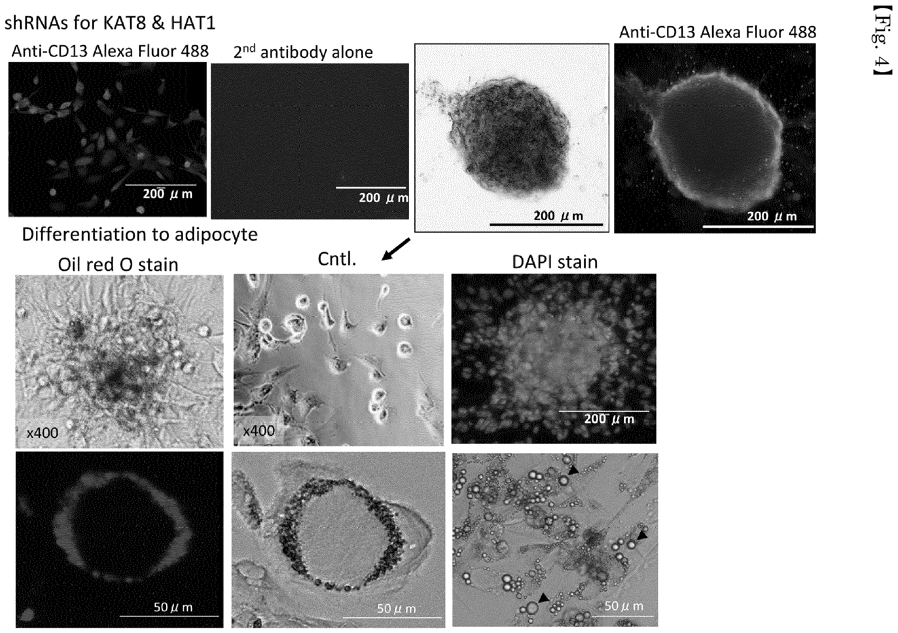

The shHAT1/shKAT8-containing HLF cells were cultured in RPMI1640 medium for 7 days and were then cultured in mesenchymal stem cell medium for additional 7 days. After that, the cells were immunostained using a hepatocyte differentiation marker CD13. FIG. 4 shows the results. The upper panels of FIG. 4 show expression of a hepatocyte differentiation marker CD13. This indicates that the cells were transformed into mesenchymal stem cells and differentiation into hepatocyte lineages were then in progress (the right upper panels). The cells were further cultured in differentiation-inducing medium (RPMI1640) for additional 7 days. Then, the significant expression of stem cell markers was not observed, and the Oil Red O staining (the left panel in the middle row) and the fluorescence analysis (the left panel in the bottom row) revealed the differentiation into cells which produced a large amount of intracellular fats. The DAPI staining (the right panel in the middle row) revealed that the spherical cells were a population of small cells. These results demonstrated that the liver cancer cells were transformed into stem cells.

### 1.5. Administration to Immuno-deficient Mice

The shHAT1/shKAT8-containing HLF cells were subcutaneously injected (200 µl; 5×10⁷ cells/mouse) into the right side of back of each of 8 KSN/Slc mice. Then, the size of the tumor was observed and recorded for two months. The size was about 10 mm at week 1 to week 3 after the transplantation. However, all of the transplanted tumors were degenerated gradually, and disappeared after two months.

### <Example 2>

### 2.1. Inhibition by Low-molecular-weight Compounds against HAT1 and KAT8

Through FDA library screening (analysis service; PLEXERA, Inc., Woodinville, WA, USA), 12 different low-molecular-weight compounds that inhibited function of both HAT1 and KAT8 were identified. The compound names are chlorpropamide, vancomycin hydrochloride, betaxolol hydrochloride, colistin sulfate, bisoprolol fumarate, pinaverium bromide, oxprenolol hydrochloride, methylbenzethonium chloride, demecarium bromide, celiprolol hydrochloride (HCl), amikacin hydrate, and alprenolol hydrochloride (hereinafter, sometimes referred to as chlorpropamide, etc.). Medicines containing each as an active ingredient (provided that each medicine has an indication other than that for malignant tumors) have already been commercially available. Because of this, these compounds seem safe.

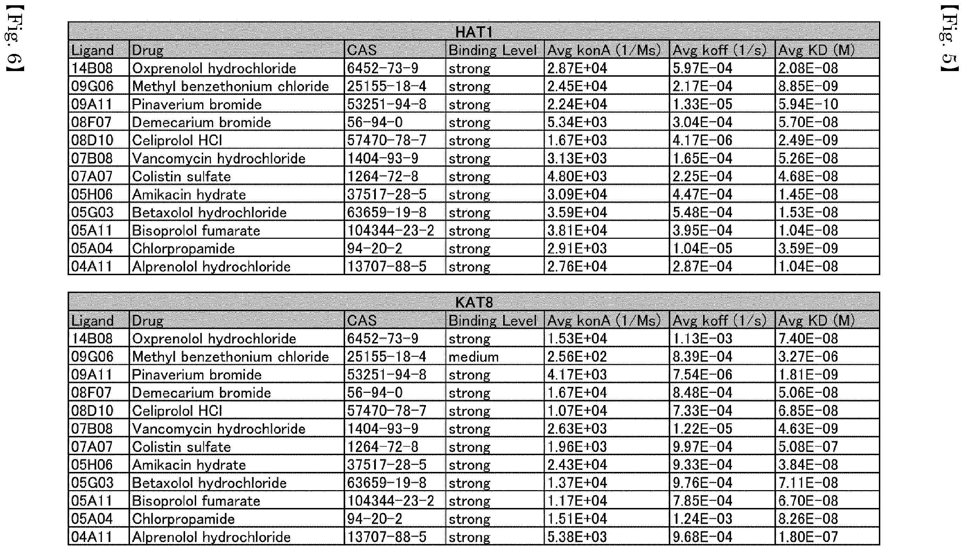

FIG. 5 shows the CAS registry number and the HAT1- and KAT8-binding strengths of each low-molecular-weight compound. Each low-molecular-weight compound had strong intermolecular binding strengths for HAT1 and KAT8.

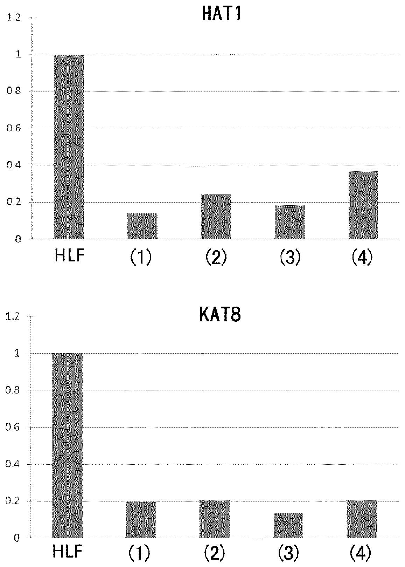

Next, the inhibitory effect of each low-molecular-weight compound on expression of HAT1 and KAT8 was examined by RT-PCR. All the chlorpropamide, etc., exerted the inhibitory effect on the expression of both HAT1 and KAT8. FIG. 6 shows the results of inhibition by each of chlorpropamide, pinaverium bromide, methylbenzethonium chloride, and colistin sulfate as representative examples. In the graph, (1) denotes chlorpropamide; (2) denotes pinaverium bromide; (3) denotes methylbenzethonium chloride; and (4) denotes colistin sulfate.

Further, through FDA library screening (analysis service; PLEXERA, Inc., Woodinville, WA, USA), 14 different low-molecular-weight compounds that inhibited only HAT1 and 5 different low-molecular-weight compounds that inhibited only KAT8 were identified. The names of the low-molecular-weight compounds that inhibited only HAT1 are fosinopril, clofilium tosylate, suprofen, esmolol hydrochloride, atractyloside potassium salt, imipenem, etifenin, cefixime, benzbromarone, cefoperazone dihydrate, pinacidil, nicergoline, oxethazaine, and antipyrine (hereinafter, sometimes referred to as fosinopril, etc.). The names of the low-molecular-weight compounds that inhibited only KAT8 are merbromin, glycopyrrolate, metoprolol-(+,-)(+)-tartrate salt, pergolide mesylate, and bumetanide (hereinafter, sometimes referred to as merbromin, etc.).

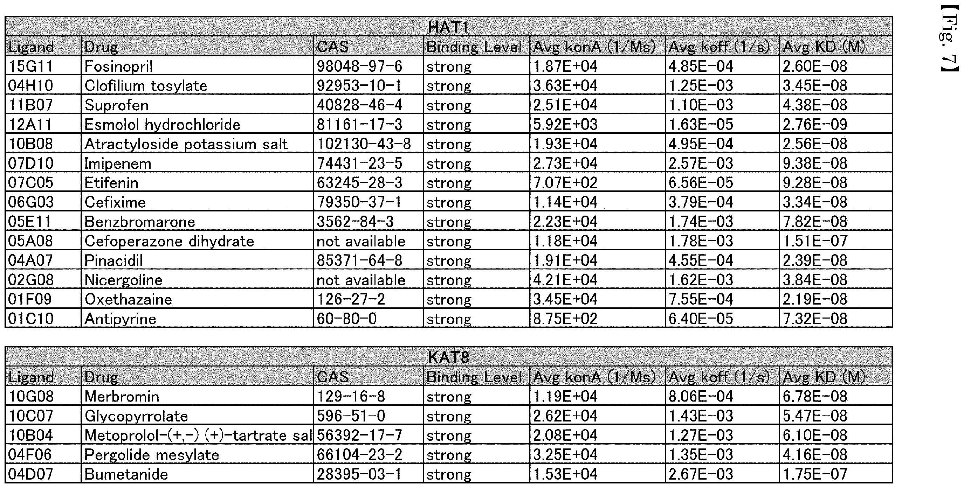

FIG. 7 shows the CAS registry number and theHAT1- or KAT8- binding strength of each low-molecular-weight compound. Each low-molecular-weight compound that inhibits only HAT1 and each low-molecular-weight compound that inhibits only KAT8 may be used in combination to inhibit function of both HAT1 and KAT8. Meanwhile, medicines containing each as an active ingredient (provided that each medicine has an indication other than that for malignant tumors) have already been commercially available. Because of this, these compounds seem safe.

### 2.2. Cell Growth Suppression

First, HLF cells were cultured on culture plates with RPMI1640 for 7 days. Next, each low-molecular-weight compound (oxprenolol hydrochloride, chlorpropamide, vancomycin hydrochloride, betaxolol hydrochloride, colistin sulfate, bisoprolol fumarate, or pinaverium bromide) was added to each culture plate. The compound concentrations after the addition were as follows (oxprenolol hydrochloride: 30 µg/µL, chlorpropamide: 30 µg/mL, vancomycin hydrochloride: 30 µg/mL, betaxolol hydrochloride: 41.8 ng/mL, colistin sulfate: 4.4 µg/mL, bisoprolol fumarate: 75 ng/mL, and pinaverium bromide: 1.0 µg/mL). Each concentration is less than or equal to the maximum effective blood concentration of a commercially available medicine containing each compound as an active ingredient (the maximum effective blood concentrations thereof are 30.18 µg/µl, 30 µg/ml, 30 µg/ml, 41.8 ng/ml, 4.4 µg/ml, 100 ng/ml, and 1 µg/ml, respectively.).

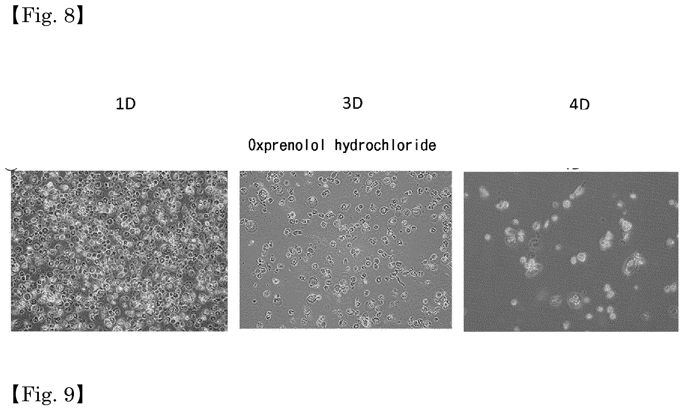

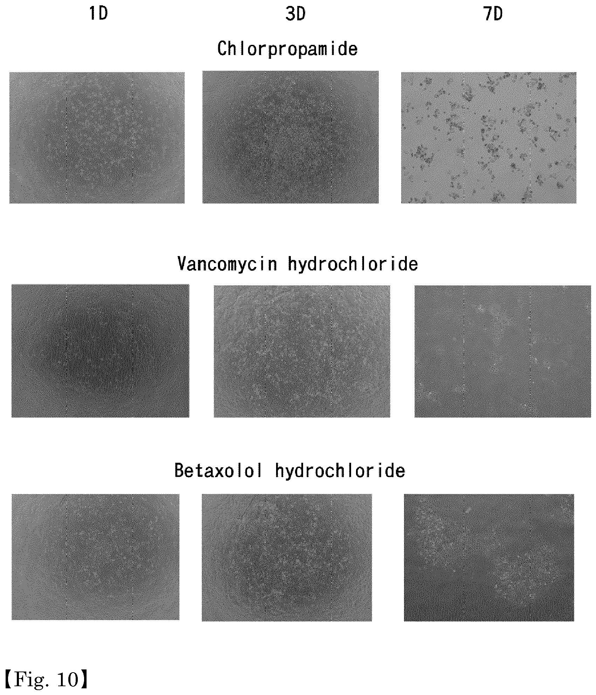

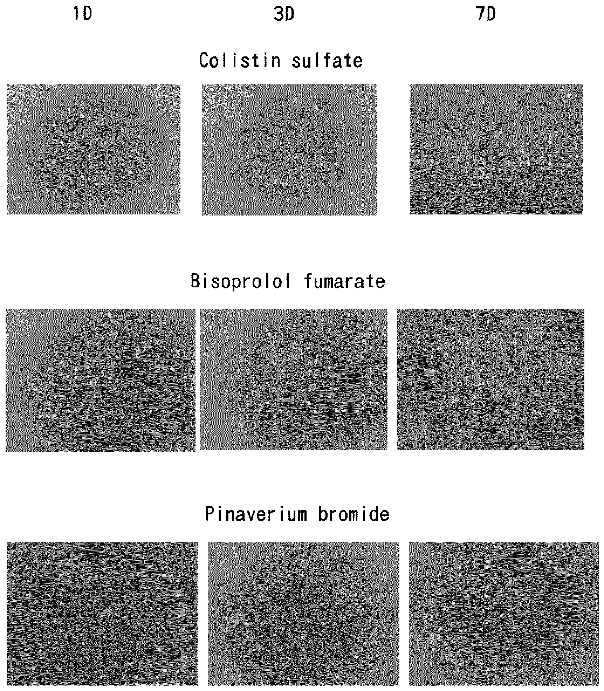

FIGS. 8 to 10 show the results at day 1, day 3, day 4, or day 7 after the addition of each low-molecular-weight compound. In the figures, the "D" denotes days. After the addition of each low-molecular-weight compound, the number of cancer cells on each plate decreased. That is, each low-molecular-weight compound within an effective blood concentration or even at the minimum effective blood concentration or less elicited a growth suppression effect on tumor cells. The results mean that each low-molecular-weight compound can be used as an effective and safe anti-cancer agent.

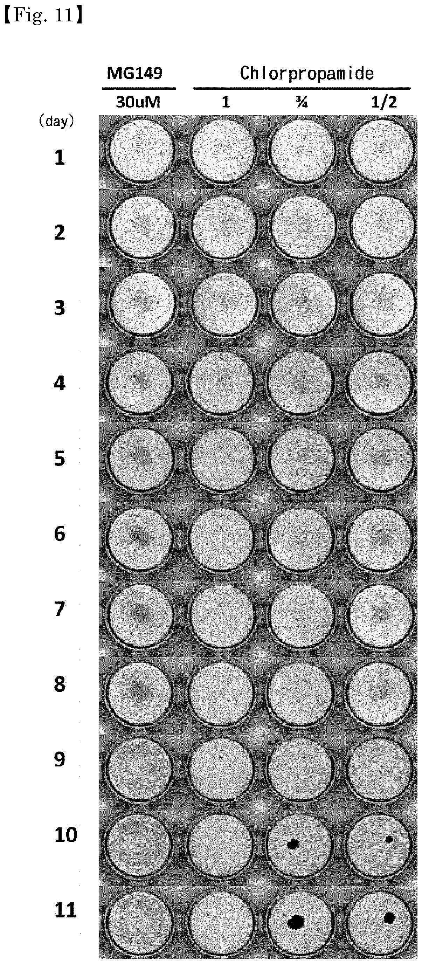

In addition, chlorpropamide or MG149 was added to HLF cells on culture plates and the cell death and cell growth suppression rate were inspected using a 3D cell culture spheroid counter Cell 3 i Mager (SCREEN, Inc., Kyoto). FIG. 11 shows the results. The rows indicate the days of culture and the columns show the kinds and concentrations of the compounds. The concentrations of chlorpropamide were represented in a ratio when the maximum effective blood concentration was set to 1. As the maximum effective blood concentration of chlorpropamide was used 30 µg/ml (which is a value described in the interview form of a commercially available medicine containing chlorpropamide as an active ingredient). MG149 is a compound that does not inhibit HAT1 but inhibits only KAT8.

The results demonstrated that MG149 at a high concentration (30 µM) failed to elicit a sufficient cell growth suppression effect. By contrast, chlorpropamide within an effective blood concentration or even at the minimum effective blood concentration or less elicited a cell growth suppression effect. The results mean that chlorpropamide can be used as an effective and safe anti-cancer agent.

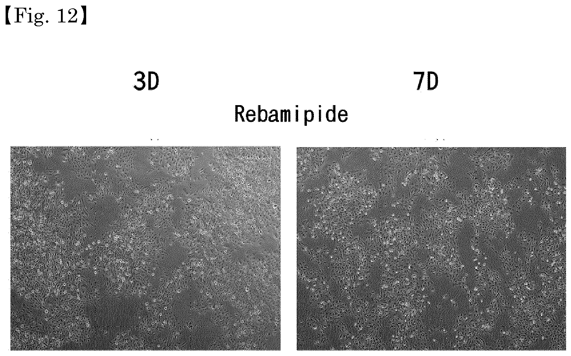

Furthermore, rebamipide was added to HLF cells on a culture pate, and the effect on the cells was examined. Rebamipide is a compound that inhibits HAT1 but does not inhibit KAT8. The rebamipide concentration after the addition was 360 µg/L. Medicines containing rebamipide as an active ingredient (provided that each medicine has an indication other than that for malignant tumors) have already been commercially available. The above concentration corresponds to the maximum effective blood concentration. FIG. 12 shows the results at day 3 and day 7. Rebamipide exhibited no cell growth suppression effect.

Moreover, how chlorpropamide exerted an anti-tumor effect on various tumors was investigated. Target tumor types, which are classified into cancer (epithelial malignant tumors), include thyroid cancer, malignant brain tumor, malignant melanoma, lung cancer, gastric cancer, liver cancer, pancreatic cancer, colon cancer, cervical cancer, ovarian cancer, bladder cancer, and neuroblastoma. Target tumor types, which are classified into sarcoma (non-epithelial malignant tumors), include fibrosarcoma and osteosarcoma.

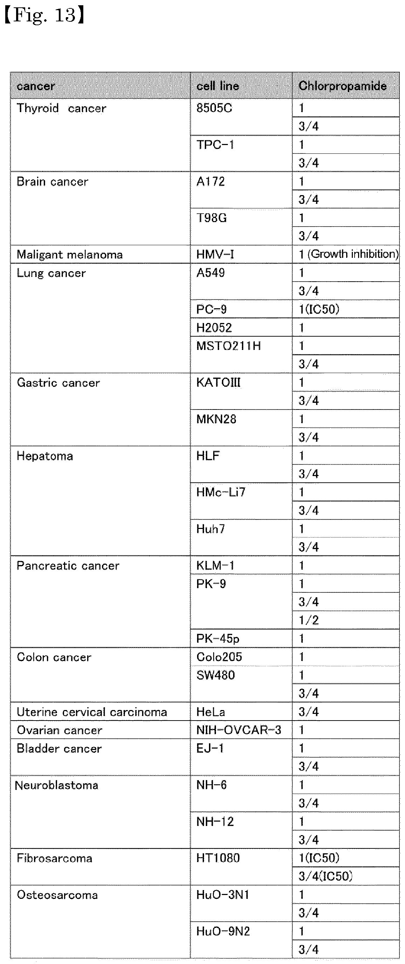

FIG. 13 shows the results. Each number represents the concentration at which an anti-cancer effect was demonstrated in a drug sensitivity test. The concentrations of chlorpropamide were represented in a ratio when the maximum effective blood concentration was set to 1 as described above. Chlorpropamide within an effective blood concentration or even at the minimum effective blood concentration or less elicited a cell growth suppression effect on each tumor. The results mean that chlorpropamide can be used as an effective and safe anti-malignant tumor agent acting on various tumors.

### <Example 3>

### 3.1. Effect of Recovering from DNA Damage

First, NHDF cells (human skin fibroblasts) were cultured on 10-cm culture plates. Next, the cells were irradiated with UV light (302 nm), which strongly causes DNA damage, for 17 min (at 0.5 J/cm²). Then, 4-min irradiation was found to be enough for cell death of the NHDF cells.

Three days after the UV irradiation, both HAT1 shRNA-expressing lentivirus particles (psi-LVRU6GP for HAT1 mixture, Genecopoeia, Inc.) and KAT8 shRNA-expressing lentivirus particles (psi-LVRU6GP for KAT8 mixture, Genecopoeia, Inc.) (hereinafter, sometimes referred to as shHAT1/shKAT8) were added to the NHDF cells on the culture plates. The additive concentration of the HAT1 shRNA-expressing lentivirus particles was 1 copy/cell and the additive concentration of the KAT8 shRNA-expressing lentivirus particles was 1 copy/cell. All virus-uninfected NHDF cells were still subject to cell death after the UV irradiation.

After about 2 weeks, it was observed that several spheroid colonies with a low level of GFP expression appeared on a plate containing the NHDF cells having the shHAT1/shKAT8. The colonies were transferred to a 6-well plate and were cultured. At 1 month or later, each spheroid colony gave rise to fibroblast-like cells. GFP expression was detected in both the spheroid colonies and the fibroblast-like cells. After trypsin treatment for cell passage, many spheroid cells with a high level of GFP expression appeared. The cells were subject to cellular senescence at average 75 days after the irradiation. The cells resulted in cell death. The above results mean that the HAT1 shRNA and KAT8 shRNA cause the cells to recover from lethal UV-induced DNA damage.

The above results have demonstrated that (i) when both HAT1 and KAT8 are inhibited, sternness characteristics of cells are induced and (ii) when only either HAT1 or KAT8 is inhibited, no sternness characteristics of cells are induced apparently. The above results have further demonstrated that (iii) when both HAT1 and KAT8 are inhibited, an excellent therapeutic effect on malignant tumors is elicited and (iv) when only either HAT1 or KAT8 is inhibited, an insufficient therapeutic effect on malignant tumors is elicited. Such results were surprising because the inhibition of both HAT1 and KAT8 exerted the effect, but the inhibition of either HAT1 or KAT8 didn't.

Besides, the above results have demonstrated that when both HAT1 and KAT8 are inhibited, cells can be recovered from DNA damage.

Hereinabove, the present invention has been described based on the Examples. These Examples are absolutely examples. It should be understood by those skilled in the art that various modifications are allowed, and those modifications are also within the scope of the present invention.

## Claims

1. A therapeutic drug for a malignant tumor, comprising a HAT1 and KAT8 inhibitor.

2. The therapeutic drug according to claim 1, wherein the inhibitor is an RNAi molecule or a low-molecular-weight compound.

3. The therapeutic drug according to claim 1 or 2, wherein the HAT1 is a HAT1 gene or protein and the KAT8 is a KAT8 gene or protein.

4. The therapeutic drug according to any one of claims 1 to 3, wherein the inhibitor is an inhibitor that inhibits expression and function of the HAT1 gene and the KAT8 gene.

5. The therapeutic drug according to any one of claims 1 to 4, wherein the inhibitor induces a sternness characteristic.

6. The therapeutic drug according to any one of claims 1 to 5, wherein the inhibitor comprises a HAT1 inhibitor and a KAT8 inhibitor.

7. A kit for treating a malignant tumor, comprising a HAT1 inhibitor and a KAT8 inhibitor.

8. A therapeutic drug for a malignant tumor, comprising a HAT1 inhibitor, wherein the HAT1 inhibitor is used in combination with a KAT8 inhibitor.

9. A therapeutic drug for a malignant tumor, comprising a KAT8 inhibitor, wherein the KAT8 inhibitor is used in combination with a HAT1 inhibitor.

10. A method for producing a stem cell, comprising the step of inhibiting HAT1 and KAT8.

11. The production method according to claim 10, wherein the inhibition is to inhibit expression and function of HAT1 and KAT8 genes.

12. A sternness characteristic inducer comprising a HAT1 and KAT8 inhibitor.

13. A sternness characteristic-inducing kit comprising a HAT1 inhibitor and a KAT8 inhibitor.

14. A sternness characteristic inducer comprising a HAT1 inhibitor, wherein the HAT1 inhibitor is used in combination with a KAT8 inhibitor.

15. A sternness characteristic inducer comprising a KAT8 inhibitor, wherein the KAT8 inhibitor is used in combination with a HAT1 inhibitor.

16. A DNA damage-ameliorating agent comprising a HAT1 and KAT8 inhibitor.

17. A DNA damage-ameliorating kit comprising a HAT1 inhibitor and a KAT8 inhibitor.

18. A DNA damage-ameliorating agent comprising a HAT1 inhibitor, wherein the HAT1 inhibitor is used in combination with a KAT8 inhibitor.

19. A DNA damage-ameliorating agent comprising a KAT8 inhibitor, wherein the KAT8 inhibitor is used in combination with a HAT1 inhibitor.
